(19) Europäisches Patentamt / European Patent Office / Office européen des brevets

(11) EP 2 407 557 B1

(12) **EUROPEAN PATENT SPECIFICATION**

(45) Date of publication and mention of the grant of the patent:
**20.05.2020 Bulletin 2020/21**

(21) Application number: **10751036.4**

(22) Date of filing: **11.03.2010**

(51) Int Cl.:
*C12Q 1/68* (2018.01)
*C12N 15/09* (2006.01)
*B03C 1/30* (2006.01)
*B03C 1/01* (2006.01)
*C12M 1/38* (2006.01)
*B03C 1/28* (2006.01)
*G01N 35/00* (2006.01)

(86) International application number:
**PCT/KR2010/001530**

(87) International publication number:
**WO 2010/104345 (16.09.2010 Gazette 2010/37)**

(54) **APPARATUS FOR INTEGRATED REAL-TIME NUCLEIC ACID ANALYSIS, AND METHOD FOR DETECTING A TARGET NUCLEIC ACID USING SAME**

VORRICHTUNG FÜR INTEGRIERTE ECHTZEIT-NUKLEINSÄUREANALYSE UND VERFAHREN ZUR ERKENNUNG EINER ZIEL-NUKLEINSÄURE DAMIT

APPAREIL POUR UNE ANALYSE D'ACIDE NUCLÉIQUE EN TEMPS RÉEL INTÉGRÉ, ET PROCÉDÉ DE DÉTECTION D'UN ACIDE NUCLÉIQUE CIBLE À L'AIDE DE CELUI-CI

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **11.03.2009 KR 20090020913**
**08.01.2010 KR 20100002027**
**10.03.2010 KR 20100021532**

(43) Date of publication of application:
**18.01.2012 Bulletin 2012/03**

(73) Proprietor: **Bioneer Corporation**
**Daejeon 306-220 (KR)**

(72) Inventors:
- **KIM, Yu-Jeong**
  **Daegu 702-022 (KR)**
- **KOO, Wan-Lim**
  **Daejeon 302-280 (KR)**
- **KIM, Jong-Hoon**
  **Daejeon 305 -509 (KR)**
- **JANG, Dae-Jin**
  **Daejeon 302-120 (KR)**
- **SEO, Jin-Cheol**
  **Daejeon 306-020 (KR)**
- **KIM, Seong-Youl**
  **Daejeon 305-506 (KR)**
- **PARK, Hae-Joon**
  **Seongnam-si**
  **Gyeonggi-do 463-010 (KR)**
- **PARK, Han Oh**
  **Daejeon 305-761 (KR)**

(74) Representative: **Hiebl, Inge Elisabeth**
**Kraus & Weisert**
**Patentanwälte PartGmbB**
**Thomas-Wimmer-Ring 15**
**80539 München (DE)**

(56) References cited:
**EP-A1- 1 674 585**
**EP-A2- 0 576 291**
**US-A1- 2001 039 014**
**US-A1- 2003 033 091**
**US-A1- 2003 124 505**
**US-A1- 2004 115 720**
**US-A1- 2004 115 720**
**US-A1- 2005 089 875**
**US-A1- 2008 003 588**
**US-A1- 2008 254 467**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 407 557 B1

SEQUENCE LISTING

<110> BIONEER CORPORATION

<120> APPARATUS FOR INTEGRATED REALTIME NUCLEIC ACID ANALYSIS, AND METHOD FOR DETECTING A TARGET NUCLEIC ACID USING SAME

<130> 23274EP

<140> 10 751 036.4
<141> 2010-03-11

<150> KR20090020913
<151> 2009-03-11

<150> KR20100021532
<151> 2010-03-10

<150> KR20100002027
<151> 2010-01-08

<160> 33

<170> PatentIn version 3.5

<210> 1
<211> 22
<212> DNA
<213> Artificial sequence

<220>
<223> Forward Primer MP1

<400> 1
agatttcagt tcaaggtcgt tc 22

<210> 2
<211> 18
<212> DNA
<213> Artificial sequence

<220>
<223> Forward Primer MP2

<400> 2
tcagttcaag gtcgttcc 18

<210> 3
<211> 18
<212> DNA
<213> Artificial sequence

<220>
<223> Forward Primer MP3

<400> 3

acaattgcag aggaggtg 18

<210> 4
<211> 18
<212> DNA
<213> Artificial sequence

<220>
<223> Forward Primer CP1

<400> 4
aagctcgaac tgtcgttc 18

<210> 5
<211> 17
<212> DNA
<213> Artificial sequence

<220>
<223> Forward Primer CP2

<400> 5
ggtgtggaaa ccttcac 17

<210> 6
<211> 19
<212> DNA
<213> Artificial sequence

<220>
<223> Forward Primer CP3

<400> 6
tgactttaag gtgtacagg 19

<210> 7
<211> 18
<212> DNA
<213> Artificial sequence

<220>
<223> Reward Primer MP1

<400> 7
gaaacccgct gacatctt 18

<210> 8
<211> 19
<212> DNA
<213> Artificial sequence

<220>
<223> Reward Primer MP2

<400> 8
gagaaagcgg acagaaacc 19

<210> 9

<211> 18
<212> DNA
<213> Artificial sequence

<220>
<223> Reward Primer MP3

<400> 9
tgggaacgac cttgaact 18

<210> 10
<211> 18
<212> DNA
<213> Artificial sequence

<220>
<223> Reward Primer CP1

<400> 10
ctaacagtgc tgtgacta 18

<210> 11
<211> 28
<212> DNA
<213> Artificial sequence

<220>
<223> Reward Primer CP2

<400> 11
caacttctat tattctattt ctagtgtc 28

<210> 12
<211> 18
<212> DNA
<213> Artificial sequence

<220>
<223> Reward Primer CP3

<400> 12
cgtctactct acgagtag 18

<210> 13
<211> 25
<212> DNA
<213> Artificial sequence

<220>
<223> TaqMan Probe (Forward) MP1

<400> 13
acgcgatgaa aaacgtctgg caagt 25

<210> 14
<211> 25
<212> DNA
<213> Artificial sequence

<220>
<223> TaqMan Probe (Forward) MP2

<400> 14
acgcgatgaa aaacgtctgg caagt 25

<210> 15
<211> 26
<212> DNA
<213> Artificial sequence

<220>
<223> TaqMan Probe (Forward) MP3

<400> 15
ctggtggaca aaaggatgga aagagc 26

<210> 16
<211> 28
<212> DNA
<213> Artificial sequence

<220>
<223> TaqMan Probe (Forward) CP1

<400> 16
agacaattca gtgaggtgtg gaaacctt 28

<210> 17
<211> 29
<212> DNA
<213> Artificial sequence

<220>
<223> TaqMan Probe (Forward) CP2

<400> 17
agtgacttta aggtgtacag gtacaatgc 29

<210> 18
<211> 27
<212> DNA
<213> Artificial sequence

<220>
<223> TaqMan Probe (Forward) CP3

<400> 18
atagaagttg aaaatcaggc gaacccc 27

<210> 19
<211> 23
<212> DNA
<213> Artificial sequence

<220>
<223> Forward Primer HBV

<400> 19
ccaatcactc accaacctct tgt 23

<210> 20
<211> 19
<212> DNA
<213> Artificial sequence

<220>
<223> Forward Primer HCV

<400> 20
gcggaaccgg tgagtacac 19

<210> 21
<211> 25
<212> DNA
<213> Artificial sequence

<220>
<223> Forward Primer CT

<400> 21
ggtattagta tttgccgctt tgagt 25

<210> 22
<211> 24
<212> DNA
<213> Artificial sequence

<220>
<223> Forward Primer NG

<400> 22
cgtaataccg catacgtctt gaga 24

<210> 23
<211> 19
<212> DNA
<213> Artificial sequence

<220>
<223> Forward Primer New H1N1

<400> 23
ggctggatcc tgggaaatc 19

<210> 24
<211> 27
<212> DNA
<213> Artificial sequence

<220>
<223> Reward Primer HBV

<400> 24
agcaggatga agaggaatat gataaaa 27

<210> 25
<211> 19
<212> DNA
<213> Artificial sequence

<220>
<223> Reward Primer HCV

<400> 25
tcaggcagta ccacaaggc 19

<210> 26
<211> 23
<212> DNA
<213> Artificial sequence

<220>
<223> Reward Primer CT

<400> 26
gtcgatcata aggcttggtt cag 23

<210> 27
<211> 23
<212> DNA
<213> Artificial sequence

<220>
<223> Reward Primer NG

<400> 27
cgccaaccag ctaatcagat atc 23

<210> 28
<211> 22
<212> DNA
<213> Artificial sequence

<220>
<223> Reward Primer New H1N1

<400> 28
tcgatgaaat ctcctgggta ac 22

<210> 29
<211> 26
<212> DNA
<213> Artificial sequence

<220>
<223> Probe HBV

<400> 29
tcctggctat cgcrggatgt gtctgc 26

<210> 30
<211> 20
<212> DNA

<213> Artificial sequence

<220>
<223> Probe HCV

<400> 30
cgtgcccccg caagactgct 20

<210> 31
<211> 25
<212> DNA
<213> Artificial sequence

<220>
<223> Probe CT

<400> 31
ctgcttcctc cttgcaagct ctgcc 25

<210> 32
<211> 22
<212> DNA
<213> Artificial sequence

<220>
<223> Probe NG

<400> 32
cttcgggcct tgcgctatcc ga 22

<210> 33
<211> 25
<212> DNA
<213> Artificial sequence

<220>
<223> Probe New H1N1

<400> 33
actctccaca gcaagctcat ggtcc 25

## Description

[Technical Field]

The present invention relates to a method for simultaneously detecting a plurality of target nucleic acids by an integrated real-time nucleic acid analysis system comprising a plurality of automated separation and purification instruments, a real-time nucleic acid amplifier, and a controller.

[Background Art]

As for in vitro diagnostic testing (IVD testing), a specific target material is detected or quantitatively analyzed by using a sample derived from a human body, such as blood, urine, saliva, or the like, as an sample, for the purpose of determining whether or not disease or infection occurs or not. Molecular diagnostic testing or nucleic acid testing (NAT) is the fastest growing field in an in vitro diagnostic testing market, but is only performed in large hospitals and clinical test specialized organizations due to complicated operations.

Qualitative and quantitative tests on virus or germs, which cause the infectious diseases, currently make up the main part of the molecular diagnostic testing, and a confirmatory test or the like is performed in order to independently confirm results of diagnostic testing on the infectious diseases or confirm mutants. The number of diagnostic tests for early identification of tumors and the increase in efficacy of therapeutic methods are rapidly increasing. The molecular diagnostic testing is variously applied in a field of personalized medicine for detecting abnormal genes, determining therapeutic methods, selecting medicine, and evaluating the efficacy of medicine, with respect to diseases caused by personal genetic predisposition.

An automated system for the molecular diagnostic testing currently used has been developed in such a manner that the whole course of extraction, amplification, identification, and the like of nucleic acid is automated. For example, as for a TIGRIS-direct tube sampling (DTS) system by Gen-Probe, which is the first automated system approved by U.S. Food and Drug Administration, capture of targets, amplification, identification, and output of test results are automated. The system can examine the infection of Neisseria gonorrhea, which is a pathogen of gonorrhea, as one of the representative venereal diseases, and Chlamydia trachomatis, which is a pathogen of a sexually transmitted disease. For another example, a COBAS system (Ampliprep, Taqman analyzer) by Roche Molecular Systems, m2000 system(m2000sp, m2000rt) by Abbott, GeneXpert system by Cepheid, Liat (Lab-in-a-tube) analyzer by IQuum Inc., have been developed in an automated system based on a polymerase chain reaction (PCR), and have been on the market.

US 2001/0039014 relates to automated devices and systems for performing nucleic acid recombination, mutation, shuffling and other diversity generating reactions in vitro, as well as related methods of performing automated diversity generation reactions. However, according to the existing automated system, since preparation of samples, real-time quantitative analysis, and report with respect to one kind of sample are performed in a single step, it is impossible to perform preparation of samples, real-time quantitative analysis and qualitative analysis, and report with respect to various different kinds of samples simultaneously and at one time.

In particular, the number of patients with hepatitis B is many while the number of patients with tuberculosis is not many. In this case, tests needs to be postponed for several days until a predetermined quantity of sample is obtained. In the case where diagnostic test is performed on several kinds of sample, a different test needs to be performed on every kind, which requires repetitive experiments, and thus, many tests cannot be performed in a day. Much time is required in collecting samples for detection of hepatitis B, and separating, purifying, and amplifying nucleic acid, in order to detect hepatitis B virus .

As such, much time is required to obtain the same kind of plural samples, and it is a waste to use a current 96-well nucleic acid amplification reactor in order to analyze only several hepatitis B samples. In this case, ultimately, an apparatus is uselessly operated or samples need to be further collected. For this reason, small-to-medium hospitals cannot perform various clinical tests on relatively small number of clinical sample due to economical and temporal limitations, and thus, outsource the tests to external test organizations, resulting in more time, more costs, and more endeavors.

### [Disclosure]

[Technical Problem]

The present inventors, while studying an automated system capable of extracting, analyzing, and reporting several kinds of targets in a single time, developed an apparatus for integrated real-time nucleic acid analysis of collecting several kinds of samples, extracting nucleic acids from the several kinds of samples on the same day, and simultaneously detecting the nucleic acids in a single nucleic acid amplifier, in order to enable small-to-medium hospitals having a relatively small number of samples to perform various diagnostic tests efficiently, and a method for analyzing a plurality of target nucleic acids using the same.

An apparatus for integrated real-time nucleic acid analysis capable of simultaneously performing qualitative analysis or quantitative analysis on genes corresponding to a plurality of various kinds of biological samples is disclosed.

An object of the present invention is to provide a method for simultaneously detecting a plurality of target a nucleic acids performed by an integrated real-time nucleic acid analysis system comprising a plurality of automated separation and purification instruments, a real-time nucleic acid amplifier, and a controller.

[Technical Solution]

An apparatus for integrated real-time nucleic acid analysis capable of simultaneously performing qualitative analysis or quantitative analysis on genes from various kinds of plural biological samples is described herein.
The present invention provides a method for simultaneously detecting-a plurality of target a nucleic acids from biological samples according to independent claim 1.

An apparatus for integrated real-time nucleic acid analysis, for simultaneously performing qualitative analysis or quantitative analysis of target nucleic acids corresponding to various kinds of biological samples is disclosed, wherein the apparatus for integrated real-time nucleic acid analysis includes: a plurality of automated purification and dispensation instruments 100 separating and purifying the target nucleic acids from the various kinds of biological samples containing the target nucleic acids; a real-time nucleic acid amplifier 200 including a multi-well temperature circulation block 210, and real-time measuring the quantity of different kinds of target nucleic acids obtained by the plurality of automated purification and dispensation instruments 100; a controller assigning multiple wells on the temperature circulation block 210 of the real-time nucleic acid amplifier 200 by the column unit, according to kinds of the target nucleic acids, correspondingly to different kinds of target nucleic acids respectively separated and purified by the automated purification and dispensation instruments 100, storing information on the biological samples from the automated purification and dispensation instruments 100 correspondingly to the respective wells assigned by the column unit, performing simultaneous amplification under the same condition of the temperature circulation block, and performing integrated management such that amplification results, by which the respective target nucleic acids are qualitatively and quantitatively analyzed, corresponds to the respective biological samples subjected to separation and purification by the automated purification and dispensation instruments 100; and a display unit 300 real-time outputting qualitative or quantitative analysis results from the controller.

In the assigning multiple wells on the temperature circulation block 210 of the real-time nucleic acid amplifier 200 by the column unit, according to kinds of the target nucleic acids, and storing information on the biological samples according to the automated purification and dispensation instruments 100 correspondingly to the respective wells assigned by the column unit, information on a positive standard sample, a negative standard sample, or a quantitative standard sample may be additively stored.

The real-time nucleic acid amplifier 200 may have diagnosis kits loaded on the multi-well temperature circulation block 210, the diagnosis kits containing different kinds of nucleic acids obtained from the plurality of automated purification and dispensation instruments 100.

The multi-well temperature circulation block 210 may be a 96-well temperature circulation block composed of 12 columns x 8 rows wells.

Measurement items may be selectively set on the multi-well temperature circulation block 210 by the column unit.

The controller, when an internal positive control (IPC) is separated together with various kinds of biological samples in each of the automated purification and dispensation instrument 100, may determine from the amplification product by the real-time nucleic acid amplifier 200 whether the nucleic acid is successfully separated by the automated purification and dispensation instrument 100, and thereby to determine whether the separation of nucleic acid is retried.

The controller qualitatively may detect the presence or absence of target nucleic acids, by comparing Ct values of respective biological samples, which are obtained through simultaneous amplification under the same condition of the real-time nucleic acid amplifier 200, with a critical Ct value.

The controller quantitatively may determine the target nucleic acid within the sample, by comparing respective Ct values obtained through simultaneous amplification of a known concentration of quantitative standard sample and respective biological samples under the same condition of the real-time nucleic acid amplifier 200, with a Ct value quantitative graph of the quantitative standard sample, to calculate the number of target nucleic acids.

The automated purification and dispensation instrument 100 may include a cartridge containing various biological samples containing nucleic acids and buffers used for extracting nucleic acids therefrom, a freezing block, a high-temperature block, a waste liquor barrel, a pipette cartridge, and a pipette block, the pipette block being movable onto a substrate on which the blocks and cartridges are provided, allowing attachment and detachment of pipettes, and including a magnetic field application unit for applying or canceling a magnetic field to the pipettes, and information on respective standard samples and biological samples and information on the target nucleic acids according to the automated purification and dispensation instruments 100 may be stored in the controller.

The real-time nucleic acid amplifier 200 may include the multi-well temperature circulation block, of which a temperature is varied according to predetermined temperature levels, an irradiation light source for irradiating light onto the reaction tubes loaded at the temperature circulation block, and a fluorescent light detection sensor for receiving lights generated from the reaction tubes, and the measurement items are set and stored by the column unit of the assigned wells.

The apparatus for integrated real-time nucleic acid analysis may further include a storage database unit 400 storing the analysis results.

According to the invention, the method for simultaneously detecting a plurality of target a nucleic acids performed by an integrated real-time nucleic acid analysis system comprising a pluarlity of automated separation and purification instruments, a real-time nucleic acid amplifier, and a controller, includes: 1) separating nucleic acids from various biological samples and standard samples and purifying the separated nucleic acids by the plurality of automated separation and purification instruments, and then 2) applying the purified nucleic acids to a plurality of reaction tubes, wherein the plurality of reaction tubes includes a first reaction tube for detecting a first target nucleic acid and a second reaction tube for detecting a second target nucleic acid different from the first target nucleic acid; 3) assigning wells of a multi-well temperature circulation block to the plurality of reaction tubes according to the target nucleic acid, wherein a first well is assigned to the second reaction tube and a second well is assigned to the second reaction tube, and storing information on the standard samples and biological samples for each well of each of the group of wells, wherein a first information on a standard sample and biological sample of the first reaction tube and a second information on a standard sample and biological sample of the second reaction tube is stored; 4) loading each of the plurality of reaction tubes prepared in step 2) to a well corresponding to the information on the biological samples, wherein the first reaction tube is loaded to the first well and the second reaction tube is loaded to the second well; and 5) simultaneously amplifying the first target nucleic acid loaded on the first well and the second target nucleic acid loaded on the second well under the same thermal condition and performing at least one of qualitative analysis and quantitative analysis on the respective target nucleic acids separated from the plurality of biological samples, thereby to obtain amplification results, wherein a first primer set and a first probe is added to the first reaction tube for amplifying and detecting the first target nucleic acid, and a second primer set and a second probe is added to the second reaction tube for amplifying and detecting the second target nucleic acid, wherein the first and second primer sets and the first and second probes are different from each other and have a similar melting point such that simultaneously amplifying and analyzing the nucleic acids are performed under the same thermal conditions, and wherein the first target nucleic acid is separated from a first biological sample and the second target nucleic acid is separated from a second biological sample different from the first biological sample.

In step 1), an internal positive control (IPC) may be added to the biological sample, followed by separation of the nucleic acid, in the automated purification and dispensation instrument 100, and the effectiveness in detection of target nucleic acid maybe determined by determining, from the amplification results, whether the target nucleic acid is successfully separated in the automated purification and dispensation instrument 100, and the amplification efficiency.

In an embodiment of the method of the invention, the reaction tube is prepared by applying the separated and purified nucleic acid solution to a reaction tube, which contains dried components necessary for amplification of nucleic acid, and the separated and purified solution and the dried components are mixed in the reaction tube.

The internal positive control may be tobacco mosaic virus particle when the target nucleic acid is RNA.

The internal positive control may be plasmid DNA or PCR product when the target nucleic acid is DNA.

[Advantageous Effects]

As set forth above, the apparatus for integrated real-time nucleic acid analysis can automatically perform separation and purification of nucleic acids from various kinds of biological samples, by using at least two automated purification and dispensation instruments, and can simultaneously analyze various samples at one time in the real-time nucleic acid amplifier, and can reduce the time and cost for test by using a controller managing the automated purification and dispensation instruments and the real-time nucleic acid amplifier.

Further, according to the method for detecting a plurality of target a nucleic acids performed by an integrated real-time nucleic acid analysis system comprising a plurality of automated separation and purification instruments, a real-time nucleic acid amplifier, and a controller, it can be confirmed whether the nucleic acids for the repective biological samples are separated or not, and whether problems occur during the nucleic acid amplificatioin procedure in the real-time nucleic acid amplifier, by adding internal positive controls of the present invention together with the biological samples in the automated purificatioin and dispensation instrument during separation and purification of nucleic acid.

Further, multiple wells of the multi-well temperature circulation block can be assigned according to the kinds of the target nucleic acids, correspondingly to different kinds of target nucleic acids separated and purified in the respective automated purification and dispensation instruments, and thus, tests of various targets required from various samples can be in parallel performed throug a single step. Therefore, the present invention can be efficeintly used by hospitals or the like needing to rapidly diagnose diseases.

[Description of Drawings]

The above and other objects, features and advantages of the present invention will become apparent from the following description of preferred embodiments given in conjunction with the accompanying drawings, in which:

FIG. 1 is a schematic view of an apparatus for integrated real-time nucleic acid analysis;
FIG. 2 is a view of a multi-well temperature circulation block 210 provided in a real-time nucleic acid amplifier 200;
FIG. 3 is a view of a display unit 300;
FIG. 4 is a view showing the entire flow diagram of the apparatus for integrated real-time nucleic acid analysis according to the present invention;
FIG. 5 is a view showing a sample loading well part of a cartridge in an automated purification and dispensation instrument;
FIG. 6 is a view showing a graph of real-time polymerase chain reactions (PCRs) of tobacco mosaic virus (TMV) standard templates in respect to tobacco mosaic virus particles (IPC), according to the present invention;
FIG. 7 is a view showing a standard curve (slope: -0.283, $R^2$:1) for the graph of the real-time PCRs of tobacco mosaic virus (TMV) standard templates from tobacco mosaic virus particles (IPC), according to the present invention;
FIG. 8 is a view showing a graph of real-time PCRs of HBV standard template DNAs in a real-time nucleic acid amplifier, using HBV quantitative PCR kits, according to the present invention;
FIG. 9 is a view showing a standard curve (slope: -0.2882, $R^2$: 0.9997) for the graph of real-time PCRs of HBV standard template DNAs in a real-time nucleic acid amplifier, using HBV quantitative PCR kits, according to the present invention;
FIG. 10 is a view showing a graph of real-time PCRs of HCV standard template RNAs in a real-time nucleic acid amplifier, using HCV quantitative RT-PCR kits, according to the present invention; and
FIG. 11 is a view showing a standard curve (slope: -0.2970, $R^2$: 0.9998) for the graph of real-time PCRs of HCV standard template RNAs in a real-time nucleic acid amplifier, using HCV quantitative RT-PCR kits.

[Detailed Description of Main Elements]

100: automated purification and dispensation instrument
200: real-time nucleic acid amplifier
300: display unit
400: storage database unit

[Best Mode]

Herein, an integrated real-time nucleic acid analysis system comprising a plurality of automated separation and purification instruments, a real-time nucleic acid amplifier, and a controller will be described in detail with reference to the accompanying drawings. The following drawings is provided by way of example so that the idea of the present invention can be sufficiently transferred to those skilled in the art to which the present invention pertains, and may be exaggeratedly drawn.

Unless indicated otherwise, it is to be understood that all the terms used in the specification including technical and scientific terms has the same meaning as those that are understood by those who skilled in the art, and further, in the description and accompanying drawings below, well-known functions or constructions will not be described in detail since they may unnecessarily obscure the understanding of the present invention.

**[Example 1] (referential)**

Example 1 is directed to an apparatus for integrated real-time nucleic acid analysis. FIG. 1 is a schematic view of an apparatus for integrated real-time nucleic acid analysis, and hereinafter, the present invention will be described in more detail.

The apparatus for integrated real-time nucleic acid analysis includes a plurality of automated purification and dispensation instruments 100 for separating and purifying different nucleic acids from various kinds of biological samples containing nucleic acids. The biological sample means a biological material separated from the natural environment and containing nucleic acid. The biological sample according to the present invention may be a nucleic acid solution, microorganism, tissue, biological fluid, or cells, which are purified or separated. Examples of the biological fluid may include blood, plasma, sputum, urine, cerebral spinal fluid, gastric lavage liquid, leukophoresis, various samples obtained from human body, and the like, but are not limited thereto. The sample of the present invention may be a sample derived from any plant, animal, human body, germ, or virus, containing nucleic acids. Examples of the nucleic acid in the present invention may generally include DNA, RNA, PNA, hybrid of DNA and RNA, or a material containing these, but are not limited thereto. The apparatus of the present invention may be used in separating and detecting a material, such as protein.

Referring to FIG. 1, automated purification and dispensation instruments 100 of the present invention may be installed in plural, preferably, 2 to 6, in order to separate nucleic acids from a single kind of, plural kinds of, or several kinds of samples. The automated purification and dispensation instrument 100 of the present invention is characterized in that a magnetic particle is reversibly bound to a nucleic acid to separate the nucleic acid, in order to separate the nucleic acids from a large number of biological sample solutions.

The automated purification and dispensation instrument 100 automatically purifies and dispenses the nucleic acids, and includes a cartridge containing various biological samples containing nucleic acids and buffers us.ed for extracting nucleic acids therefrom, a freezing block, a high-temperature block, a waste liquor barrel, a pipette cartridge, and a pipette block. The pipette block can move onto a substrate in which the above blocks and cartridges are provided, allowing attachment and detachment of pipettes, and including a magnetic field application unit for applying or canceling a magnetic field to the pipettes. The pipette block can allow attachment and detachment of 8 to 16 pipettes.

The magnetic particles used in separating the nucleic acids from a large number of biological sample solutions bind to the nucleic acids in a buffer. If a magnetic field is applied to the pipette while the nucleic acids bound to the magnetic particles are sucked in the pipette, the magnetic particles bound to the nucleic acids agglomerate in the pipette and adhere to an inside of the pipette, and the other materials are discharged out of the pipette.

As the magnetic particle of the present invention, a fine magnetic particle having a wide surface area is preferably used.

In the present invention, a permanent magnet or an electromagnet may be used as the magnetic field application unit for applying the magnetic field to the pipette, and the magnetic field may be reversibly applied to the pipette. The solution may be discharged by a piston connected to the pipette during operation of the pipette, and the pipette is movable by a pipette moving unit.

As the automated purification and dispensation instrument 100 of the present invention, a commonly known automated purification and dispensation apparatus may be used, and for example, an apparatus described in Korean Patent Registration No. 148239, US5702590, US5647994, EP 0691541, US 5336760, US5897783, US6187270, and Korean Patent Application No. 10-2008-0032904 may be used. Also, ExiPrep™ 16-fully Automated DNA/RNA/Proteins Purification System by Bioneer Company may be preferably used as the automated purification and dispensation instrument 100 of the present invention.

The nucleic acid separated and purified by the automated purification and dispensation instrument 100 is dispensed in diagnosis kit constituted of a unit reaction tube previously loaded therein. The diagnosis kit constituted of an 8-well strip tube, in which the nucleic acid is dispensed in the automated purification and dispensation instrument 100, is taken out, and sealed with a transparent film, and then loaded in the column assigned in a real-time nucleic acid amplifier. An operation of the real-time nucleic acid amplifier is run, and thus, the results are automatically drawn.

A bar code recognition unit (not shown) may be added in the plurality of automated purification and dispensation instruments 100 for separating and purifying nucleic acids from the biological samples containing the nucleic acids. Biological sample information recognized by the bar code recognition unit may be stored as database in a controller, which is system manager software within the apparatus for integrated real-time nucleic acid analysis, and this information may be automatically applied also in the real-time nucleic acid amplifier 200. The bar code exhibits contents necessary for distinguishing among samples, such as sex, age, kinds, and the like of the biological samples.

The present invention includes a real-time nucleic acid amplifier 200 of amplifying different kinds of target nucleic acids obtained from the plurality of automated purification and dispensation instruments 100 in real time.

The real-time nucleic acid amplifier 200 of the present invention includes a multi-well temperature circulation block 210, in which diagnosis kits corresponding to the nucleic acids separated and purified from the automated purification and dispensation instruments 100 are loaded, and of which a temperature is varied according to predetermined temperature levels, a light source for irradiating light into the reaction tubes loaded at the temperature circulation block, and a fluorescent light detection sensor for receiving light generated from detectable labels within the reaction tubes. The fluorescent light detection sensor allows the real-time qualitative and quantitative analysis results with respect to the target nucleic acids.

As the real-time nucleic acid amplifier 200 of the present invention, which is an apparatus allowing real-time PCR, a known apparatus for real-time nucleic acid polymerase chain reaction (PCR) may be used, and preferably, an apparatus described in Korean Patent No. 794703, an apparatus described in PCT/KR2008/064558, or Exicycler™ 96 Real-Time Quantitative Thermal Block, a product by Bioneer Company, may be selected and used.

The real-time nucleic acid amplifier 200 includes the multi-well temperature circulation block 210. The multi-well temperature circulation block 210 is composed of 96 wells of 12 columns x 8 rows. Respective wells are partitioned by the column unit, and thereby assigned by the column unit. Thus, amplification quantity of respective nucleic acids and target information according to different kinds of target nucleic acids can be displayed by the column unit through the display unit 300 in real time. In the present example, detection items of target nucleic acids are set by the column unit of wells, but, without the limitation thereto, they may be set by the row unit or by the well unit.

In the present invention, the apparatus for integrated real-time nucleic acid analysis may further include a storage database unit 400 storing analysis results.

The real-time nucleic acid amplifier 200 of the present invention stores information on the kinds of target nucleic acids separated and purified by the automated purification and dispensation instruments 100 and the biological samples. Also, the real-time nucleic acid amplifier 200 assigns multiple wells on the multi-well temperature circulation block 210 by the column unit, according to the kinds of respective target nucleic acids separated from the respective automated purification and dispensation instruments 100. One or more columns are preferably assigned on the multi-well temperature circulation block. The nucleic acids separated from the biological sample and the standard samples are put into respective wells within each of the assigned area that is set to have the same detection target, and related information is stored for every well.

In the present invention, a 'well 211a' means each well of the multi-well temperature circulation block 210 on which a reaction tube of the diagnosis kit can be loaded. Respective detection targets are set by the column unit on the multi-well temperature circulation block of the nucleic acid amplifier according to the kinds of the diagnosis kits.

The present invention includes a real-time nucleic acid analysis integrated apparatus manager software controlling the automated purification and dispensation instruments 100, controlling the real-time nucleic acid amplifier 200, transmitting the information from the automated purification and dispensation apparatus to the nucleic acid amplifier in real time, and storing and managing the information of the real-time nucleic acid amplifier.

The diagnosis kit used in the present invention includes a detectable label, and is composed of reaction tubes 500 in which a primer for amplifying a specific target nucleic acid, and a buffer are contained in the same quantity.

In the present invention, the 'reaction tube 500' means a container for containing a sample therein. 1 to 96, preferably 8, 12, 16, or 96 reaction tubes may be prepared. The reaction tube may be a dent or a tube formed in a plastic or similar material, and may be a tube having a regular pattern, for example, a lattice type of 96-well reaction plate or 384-well reaction plate, or 8-well strip type.

A primer for amplifying the target nucleic acid, a detectable label, and a buffer are contained in the reaction tube 500 for the diagnosis kit, and the separated nucleic acid is added thereto. The total volume thereof is preferably 10 to 50 $\mu\ell$, but not limited thereto. Different primers and probes in several diagnosis kits are amplified at the same annealing temperature ($T_1$). Each unit reaction tube of the diagnosis kit contains a primer and a probe designed to amplify a different target nucleic acid.

In the present invention, the diagnosis kit is prepared by drying a composition in which a specific primer for amplifying a specific part of a nucleic acid, a probe having fluorescence attached thereon, a DNA polymerase, and dNTP are mixed. A stabilizer may be added therein. The drying may be performed by Freeze-drying, heated drying, vaccuum drying under reduced pressure or the like. Therefore, since the composition of the diagnosis kit is present in a dried type, a predetermined quantity of separated and purified nucleic acid solution is added thereto, without regulation of the quantity of the composition, and thereby to directly prepare a nucleic acid amplification reaction ' mixture.

The primers for detecting the target nucleic acids, which used in respective diagnosis kits in the present invention, use base sequences designed to have similar melting points (Tm, °C) for diagnostic test of respective target nucleic acids. The melting point (Tm, °C) is designed based on a part of the base sequence of the target nucleic acid, and it is important to set the same reaction temperature condition in order to simultaneously and selectively amplify various target nucleic acids on the base of an annealing temperature. According to the present invention, several target nucleic acids can be simultaneously detected from samples from one person or samples from several persons. For example, tests for diagnosing AIDS, hepatitis B, hepatitis C, and venereal diseases can be performed on a single real-time nucleic acid amplifier under the same amplification condition at one time.

FIG. 2 is a view of a multi-well temperature circulation block 210 having a plurality of wells in which the plurality of reaction tubes 500 for diagnosis kits are loaded, respectively. In the present invention, the reaction tube 500 may be made in an 8-strip type, a 16-strip type, or 96-well plate type.

Referring to FIG. 2, reaction tubes, in which the nucleic acids separated and purified from the same kind of biological samples are injected, are loaded in the wells 211a on the assigned columns, and the primer and/or the probe designed to amplify the same target nucleic acid are contained in each of the reaction tubes loaded on the same columns.

Referring to FIG. 2, the multi-well temperature circulation block 210 is composed of a plurality of wells 211. Reaction tubes 500 for the diagnosis kits may be loaded in the respective wells 211a. In the present invention, several different reaction tubes for the diagnosis kits may be loaded onthemulti-well temperature circulation block, and the multiple wells of the multi-well temperature circulation block are assigned by the column unit, according to the kinds of target nucleic acids. A plurality of diagnosis kits for different target nucleic acids are loaded on the multi-well temperature circulation block, and thus, the multiple wells on the multi-well temperature circulation block are assigned for the respective kinds of target nucleic acids by the column unit, correspondingly to different kinds of target nucleic acids. However, diagnostic tests are performed under the same amplification reaction condition.

The multi-well temperature circulation block 210 of the real-time nucleic acid amplifier of the present invention may have a 96-well (12 columns x 8 rows) type. The multiple wells on the multi-well temperature circulation block are preferably set to be assigned for detection targets by the unit of adjacent one or two columns, but not limited thereto.

The real-time nucleic acid amplifier 200 of the present inventionhas a temperature, which is repetitively variedbetween a first temperature as an annealing temperature ($T_1$) and a second temperature as an expansion temperature ($T_2$), under control of the controller.

Therefore, the reaction tubes for diagnosis kits loaded in the wells 211a have a temperature between the first temperature as an annealing temperature ($T_1$) and the second temperature as an expansion temperature ($T_2$), and thus, the nucleic acids are amplified, and a third temperature may be required for denaturation. The first temperature as an annealing temperature ($T_1$), the second temperature as an expansion temperature ($T_2$), and the third temperature of the multi-well temperature circulation block 210 may be variously set depending on the kind of the target nucleic acid.

The real-time nucleic acid amplifier 200 measures the amplification degrees and quantities of respective target nucleic acids simultaneously, based on information on the kinds of target nucleic acids and the biological samples corresponding to the wells assigned on the multi-well temperature circulation block. The target nucleic acids are measured by numerical values of signals of detectable labels contained in the reaction tubes.

The level of the signal generated by the label is proportional to the quantity of target nucleic acids amplified. The signal of the detectable label may be any kind of signal including, for example, a luminescent signal, a color dye signal, or a radioactive signal. The detectable signal is preferably the luminescent signal. The luminescent signal may be a fluorescent signal or a chemiluminescent signal. The light is illuminated as the amplification of target nucleic acid is started, and the illuminating time and the illumination intensity are varied depending on the amplification quantity of the target nucleic acid. These enable monitoring of the amplification quantity of the target nucleic acid.

In the present invention, a probe bound with a fluorescent die may be used for detection of signals, and a fluorescent die, such as an intercalating die, may be used. Preferably, a dual-labeled probe may be mainly used for amplification of nucleic acid.

The real-time nucleic acid amplifier 200 includes a controller. The controller stores information about the kinds of target nucleic acids separated by the automated purification and dispensation instruments 100 and respective biological samples separated and purified by the automated purification and dispensation instruments 100, assigns multiple wells for the respective kinds of target nucleic acids correspondingly to different kinds of target nucleic acids separated and purified by the automated purification and dispensation instruments 100, simultaneously amplifies all the target nucleic acids on.the multi-well temperature circulation block under the same condition, and measures and determines the respective target nucleic acids which are simultaneously amplified, based on information about the kinds of target nucleic acids corresponding to the assigned columns and the biological samples assigned for the respective wells.

All the information maintained by the controller is inputted from a user, and the inputted information is set to be stored and maintained.

In this real-time nucleic acid amplifier 200, the amplification quantity of respective target nucleic acids and target information are displayed through the display unit 300 in real time, according to the kinds of diagnosis kits for detecting different target nucleic acids, which are loaded on the multi-well temperature circulation block 210 by the column unit or by the well unit.

FIG. 3 is a view of a display unit 300. Referring to FIG. 3, the display unit 300 may include a first display unit 302 of displaying information on the assigned columns of the real-time nucleic acid amplifier 200, a second display unit 301 of measuring and graphing the quantity of target nucleic acid amplified in the unit reaction tube, and a well selector 303 of enabling a user to individually select and view the wells 211a. By selection of the user through the well selector 303, diagnostic results for one or more unit reaction tubes are displayed in different colors at the same time, and thus, differences in the diagnostic results can be clearly and distinguishably displayed.

The apparatus for integrated real-time nucleic acid analysis includes a controller for integratedly managing the automated purification and dispensation instruments and the real-time nucleic acid amplifier, and more specifically, for transmitting data of the biological samples from the automated purification and dispensation instruments to the real-time nucleic acid amplifier, and qualitatively or quantitatively analyzing target nucleic acid information and amplification results of different kinds of target nucleic acids, which are obtained from the real-time nucleic acid amplifier.

The integrated apparatus for real-time nucleic acid analysis is operated by a software of the controller, which is divided into four, that is, 1) input of information, 2) separation and purification of nucleic acid in the automated purification and dispensation instrument 100, 3) run of the real-time nucleic acid amplifier 200, and 4) update result and analysis, as shown in FIG. 6. The software of the controller of the present invention controls the entire operation. The software performs DB function and maintenance, control and maintenance of the automated purification and dispensation instruments, run, analysis, control and maintenance of the real-time nucleic acid amplifier, and the like.

The apparatus for integrated real-time nucleic acid analysis is composed of two computers, three automated purification and dispensation instruments, and one real-time nucleic acid amplifier. First, the software of the controller is based on data inputted to an integrated real-time nucleic acid analysis apparatus manager software, by a user. One of the two computers is a computer for the integrated real-time nucleic acid analysis apparatus manager software on which a local DB, an integrated real-time nucleic acid systemmanager software, and an automated purification and dispensation instrument program are installed. The other computer is for a real-time nucleic acid amplifier program. Respective computers transmit and receive data through TCP/IP. The computer for the integrated real-time nucleic acid analysis manager software communicates with the tree automated purification and dispensation instruments through TCP/IP. The computer for the real-time nucleic acid amplifier communicates with the real-time nucleic acid amplifier through a USB. The apparatus for integrated real-time nucleic acid analysis is constituted of a client server environment among the computers, and a client server environment between the integrated real-time nucleic acid analysis apparatus manager software and the automated purification and dispensation instruments.

More specifically, a main DB is present at each hospital site. Data related to samples are inputted to the main DB by the integrated real-time nucleic acid analysis apparatus manager program. Through this work, a work list is provided on the local DB used in the apparatus for integrated real-time nucleic acid analysis. The provided work list is assigned to respective' automated purification and dispensation instruments using a bar code or automatic assignment type by the user. Upon completion of assignment is completed, the automated purification and dispensation instruments are operated. Upon completion of an operation, updating following the completion of an operation is performed. The computer for the integrated real-time nucleic acid analysis apparatus manager software checks for updating, and thereby completes the updating on the local DB. The integrated real-time nucleic acid analysis manager software of the present invention can control several nucleic acid purification and dispensation instruments simultaneously and individually, and manage operation state thereof.

The computer for the integrated real-time nucleic acid apparatus manager software notifies the user that the real-time nucleic acid amplifier is ready. Reaction tubes are loaded on a 96-well type multi-well temperature circulation block, after receipt of the notification, by the user. When the loading of the reaction tubes is completed, the real-time nucleic acid amplifier is run. Upon completion of the run, the computer for the integrated real-time nucleic acid apparatus manager software notifies the user, whether or not an analysis program is run. On completion of the analysis, completion and ending are performed by the user. The computer for the integrated real-time nucleic acid apparatus manager software checks that all operations and analysis are completed. The local DB is updated after completion of the checking.

First, data information needs to be inputted in order to operate the apparatus for integrated real-time nucleic acid analysis of the present invention. The input of data information means that the user inputs the data information according to the sample in conformity to the database within the integrated manager program of the computer for the integrated real-time nucleic acid apparatus manager software. When data information is inputted, protocols used according to the diagnosis kits are defined. In other words, a name of a sample is inputted, and a diagnosis kit, a nucleic acid type, a sample source type, and a separation protocol are selected. The number of samples to be inputted is selected, and then the 'input' button is pressed. In the automated purification and dispensation instrument, cartridge wells are respectively 'assigned' by using the inputted data information. In the present invention, 'auto assignment' and bar code 'assignment' are supported as the 'assignment'. The 'assignment' is automatically performed in an order in which the samples to be purified are clicked. When the 'assignment' is completed, each of the automated purification and dispensation instruments is in an operable state. Respective units and instruments of the automated purification and dispensation instrument are operated by the-user . The automatic purification and dispensation instrument software stores all the information related to the operation. The automatic purification and dispensation instrument software provides progress information on the time, the progress state, and the like, at the time of operation to the user. The operation time of the automated purification and dispensation instrument is about 40 to 50 minutes. When the operation of the automated purification and dispensation instrument is completed, the integrated real-time nucleic acid analysis apparatus manager software performs automatic detection. Following the automatic detection, an operation of the real-time nucleic acid amplifier, which is a next process, is instructed.

In the present example of the present invention, a corresponding computer for the real-time nucleic acid amplifier is separately defined, and thus, the user makes the real-time nucleic acid amplifier program run. The work list is transferred from the automated purification and dispensation instrument to the real-time nucleic acid amplifier, and thereby, to be automatically provided to the user. Diagnosis kits to be experimented are selected by the nucleic acid amplifier program run through the integrated real-time nucleic acid analysis manager software. In the present invention, controls are automatically assigned depending on the selected diagnosis kit. Work is performed by the user according to the work list performed by the automated nucleic acid purification and dispensation instrument. The multiple wells are 'assigned' in conformity to the order in which the samples are clicked. Diagnosis kits are loaded in conformity to the corresponding information. The real-time nucleic acid amplifier is operated. The operation of the real-time nucleic acid amplifier takes about 2 to 3 hours. When the operation s/w of the real-time nucleic acid amplifier is finished, a corresponding result file is transmitted to the manager software. When the operation is completed, the operation state is automatically recognized, the results are updated, and the work list is transferred to a next process, by the integrated real-time nucleic acid analysis apparatus ’manager’ software. All results are updated by performing updating of the results through the ’manager’ program. Samples to be updated are selected according to the wells of the multiple wells. Updating is not performed with respect to failures of the samples, which are returned to a corresponding process, such as a separation process or an amplification process. The ’Analysis’ window for showing all current analysis is displayed, which enables the user to analyze the amplification sate.

The controller of the apparatus for integrated real-time nucleic acid analysis of the present invention automatically connects five programs, that is, the ’work list’ local database, the integrated real-time nucleic acid analysis apparatus manager software, the automated purification and dispensation apparatus software, the real-time nucleic acid amplifier operation software, and the real-time nucleic acid amplifier analysis software, and thus provides a series of automatic process. A screen relating assignment is provided at the time of ’assignment’ in order to prevent human errors, and all the data are traceable. Log files are created at the time of operating all units and instruments.

The apparatus for integrated real-time nucleic acid analysis may further include a storage database unit 400 storing analysis results of the unit reaction tubes of respective diagnosis kits.

**[Example 2]**

Example 2 is directed to a method for detecting target a nucleic acid using the apparatus for integrated real-time nucleic acid analysis as disclosed above, and hereinafter, this will be described in detail.

The present invention provides a method for detecting a plurality of target a nucleic acids performed by an integrated real-time nucleic acid analysis system comprising a plurality of automated separation and purification instruments, a real-time nucleic acid amplifier, and a controller, the method including:

1) separating nucleic acids from various biological samples and standard samples and purifying the separated nucleic acids by the plurality of automated separation and purification instruments;
2) applying the purified nucleic acids to a plurality of reaction tubes, wherein the reaction tube includes a first reaction tube for detecting a second target nucleic acid different from the first target nucleic acid;
3) assigning wells of a multi-well temperature circulation block to the plurality of reaction tubes according to the target nucleic acid, wherein the first well is assigned to the first reaction tube and a second well is assigned to the second reaction tube, and storing information on the standard samples and biological samples for each well in a group of wells, wherein a first information on a standard sample and biological sample of the first reaction tube and a second information on a standard sample and biological sample of the second reaction tube is stored;
4) loading each of the plurality of reaction tubes prepared in step 2) to a well corresponding to the information on the biological samples, wherein the first reaction tube is loaded to the first well and the second reaction tube is loaded ot the second well; and
5) simultaneously amplifying the first target nucleic acid loaded on the first well and the second target nucleic acid loaded on the second well under a same thermal condition and performing at least one of qualitative analysis and quantitative analysis on the respective target nucleic acids separated from the plurality of biological samples, thereby to obtain amplification results, wherein a first primer set and a first probe is added to the first reaction tube for amplifying and detecting the first target nucleic acid, and a second primer set and a second probe is added to the second reaction tube for amplifying and detecting the second target nucleic acid, wherein the first and second primer sets and the first and second probes are different from each other and have a similar melting point such that simultaneously amplifying and analyzing the nucleic acids performed under the same thermal conditions, and wherein the first target nucleic acid is separated from a first biological sample and the second target nucleic acid is separated from a second biological sample different from the first biological sample.

In the present invention, step 3) and step 4) may be simultaneously performed, or step 4) may be performed before step 3).

In the present invention, the nucleic acids purified and dispensed from the automated purification and dispensation instruments 100 may be different nucleic acids, or several kinds of biological samples may be used in separation of nucleic acids in step 1).

In the present invention, an internal positive control (IPC) is added to the biological sample, followed by separation of the nucleic acid, in the automated purification and dispensation instrument 100, and the effectiveness in detection of target nucleic acid is determined by determining, from the amplification results, whether the target nucleic acid is successfully separated in the automated purification and dispensation instrument 100, and the amplification efficiency.

In the present invention, in step 2), the reaction tube is prepared by applying the separated and purified nucleic acid solution to a reaction tube, which contains dried component necessary for amplification of nucleic acid, and the separated and purified solution and the dried components are mixed in the reaction tube.

Since described the diagnosis kit is made of a dry composition, the separated and purified nucleic acid solution is added thereto, and thereby to directly prepare a mixture. When the separated nucleic acid solution is added to the diagnosis kit in a solution state, the reactive volume is increased and the concentration of the separated nucleic acid is decreased.

In the present invention, the internal positive control is tobacco mosaic virus particle when the target nucleic acid is RNA.

In the present invention, the internal positive control is plasmid DNA or PCR product when the target nucleic acid is DNA. The PCR amplification product means DNA made by PCR amplification.

In step 1), the internal positive control may further include quantitative standard samples which are added according to the known concentrations.

In step 1), a negative control (NTC) and a positive control (PC) may be further used as the standard samples.

As the primer and the probe for amplification of the internal positive control, sequences represented by Table 1 below may be used.

In the present invention, the internal control (or internal positive control, IPC) is applied from a nucleic acid separation and purification step, and amplification is performed in the real-time nucleic acid amplifier after the nucleic acid is separated from the sample. This is used for solving the problem in that it is difficult to confirm whether the cause of insufficient amplification of nucleic acid lies in a purification procedure or an amplification procedure. In the present invention, the internal positive control (IPC) having a known concentration is added to the sample from the separation procedure, and separated and purified together with the nucleic acid, and thus, it can be confirmed whether separation is properly performed in the automated nucleic acid purification and dispensation instrument or not as well as it can easily be seen whether the cause of insufficient amplification of nucleic acid during the amplification procedure after separation lies in the separation step or in the amplification step. In the present invention, a tobacco mosaic virus is used as the internal positive control (IPC) . The tobacco mosaic virus (TMV), which is an RNA virus, may be properly used as the internal positive control (IPC) when an RNA virus, such as influenza A virus subtype H1N1, is a target for detection.

Advantages of using the tobacco mosaic virus (TMV) are as follows. In the prior art, the internal positive control (IPC) is not used together with the nucleic acid when the nucleic acid is to be separated from the sample, and a specific kind of RNA is added to the separated nucleic acid sample only at the time of nucleic acid amplification reaction. However, in the present invention, the virus particles themselves are subjected to separation, purification, and amplification, together with the nucleic acid, from the separation step of sample to real-time nucleic acid amplification reaction, and thus they are useful in assay of efficiency. The tobacco mosaic virus (TMV), which is a plant virus, allows mass purification and does not accompany risk on treatment. Since a human virus and a plant virus have different reactive 'receptors', human is not infected by the plant virus. Also, since the plant virus has a far relationship with the human virus, a base sequence thereof has a little or little concordance with a base sequence of a target nucleic acid of human at the time of preparing a primer and a probe or at the time of nucleic acid amplification reaction.

The description also relates to a diagnosis kit which is composed of an internal positive control (IPC), a primer corresponding to the amplified target nucleic acid, a detectable label for detecting the target nucleic acid, and a buffer, which are provided in the same quantity within a unit reaction tube. As the unit reaction tube, an 8-well strip may be used. Also, the reactants for nucleic acid amplification, within the unit reaction tube, are characterized to be made in a dry type.

In the present invention, the amplification result, from which the internal positive control (IPC) is added to the biological sample to separate and identify the nucleic acid in the real-time nucleic acid amplifier 200, can be confirmed from the separation or non-separation of the nucleic acid obtained from the automated purification and dispensation instrument 100 and the amplification procedure of performing simultaneous amplification under the same condition, using the real-time nucleic acid amplifier 200.

The herein disclosed apparatus for integrated real-time nucleic acid analysis can simultaneously amplify the nucleic acids separated from the plurality of automated purification and dispensation instruments at one time through the real-time nucleic acid amplifier 200.

Also, the description relates to collecting information on target nucleic acids to be detected from the respective simultaneously amplified nucleic acids.

For example, nucleic acids may be separated from blood, urine, sputum, cells, and the like through different procedures, but the nucleic acids separated therefrom can be simultaneously amplified in a single amplifier. In the present invention, the plurality of nucleic acids are separated by several automated purification and dispensation instruments. The nucleic acids are respectively separated from blood, urine, sputum and sputum samples by the tree automated purification and dispensation instruments. The separated nucleic acids may be applied in simultaneously detecting AIDS, hepatitis B, hepatitis C, malaria, tuberculosis, and venereal disease, using the diagnosis kits therefore.

In order to simultaneously detect causes of these diseases by the real-time nucleic acid amplifier, the multiple wells on multi-well temperature circulation block may be partitioned by the column unit, and thereby to assign a specific well for detection. For example, columns of the multi-well temperature circulation block are assigned such that a first column is assigned for AIDS, a second column for hepatitis B, a third column for hepatitis C, and a fourth column for venereal disease, and then the amplification conditions thereof are set in the same manner. Then, diagnosis kits containing the separated nucleic acids are respectively loaded on the assigned wells on the multi-well temperature circulation block, and then the amplification reaction is performed. In order to set the amplification reaction conditions in the same manner, Tm values of the reaction conditions of respective diagnosis kits are set in the same manner or similarly. Simultaneous amplification is performed in the 96-well nucleic acid amplifier under the same reaction condition of PCR in which the primer or probe is set to have the same Tm value. As the result, convenience can be improved and the time for detection can be shortened. Above all, time error, which is caused by sequentially detecting several kinds of targets one by one, can be reduced. Also, an identification procedure can be easily performed since a plurality of target DNAs or RNAs are simultaneously detected under the same condition by the real-time nucleic acid amplifier instead of detecting only one kind of target pathogen at a single time. As such, several pathogens can be simultaneously identified in a short time, by separating nucleic acids from several kinds of samples derived from one person or samples derived from several persons and simultaneously detecting various target nucleic acids therefrom at one time. In the present invention, test items of a specific pathogen are assigned by the column unit of the multi-well temperature circulation block.

A procedure of separating and purifying the nucleic acids from the biological samples is further disclosed, using the plurality of automated purification and dispensation instruments 100 is as follows . In the present exemplary variant, ExiPrep™ Fully automated nucleic acid extraction instrument by Bioneer Company was adopted and used as an automated nucleic acid purification instruments, and ExiPrep™ extraction kit by Bioneer Company was modified and used as a nucleic acid purification kit.

The nucleic acid purification kit of the automated purification and dispensation instrument is composed of a cartridge set in which buffers used in extracting nucleic acids from various biological samples containing nucleic acids are included, and a plastic consumable set used in purification. The cartridge set is composed of cartridge I and cartridge II. The cartridge I contains protease for intracellularproteolysis, a lysing solution for cell lysis, silica magnetic particles for binding the eluted nucleic acid, and a binding solution for binding the eluted nucleic acid and the silica magnetic particles. The cartridge II contains at least one kind of washing solution for washing materials except nucleic acid attached on surfaces of the silica magnetic particles and an eluting solution for efficiently separating nucleic acids from the surfaces of the silica magnetic particles. The plastic consumable set is composed of a disposable filter tip used in transporting and transferring various kinds of buffers, a reaction tube used in a cell lysis procedure by the protease, and an elution tube for storing the finally purified nucleic acid.

The cartridge of the nucleic acid purification kit is a multi-well cartridge, and a 96-well cartridge may be preferably used. ExiPrep™ viral DNA/RNA kit by Bioneer Company may be adopted and used as the nucleic acid purification kit for separating New influenza A(H1N1) RNA. A viral RNA extraction procedure is composed of a sample pretreatment procedure, an addition procedure of a positive control, a negative control, an internal positive control (IPC), and sample, and a nucleic acid extraction procedure using the automated purification and dispensation instrument. Since the extracted nucleic acid is automatically mixed with a diagnosis kit for real-time RT-PCR, viral RNA needs to be manually inputted in the diagnosis kit, after extraction of the nucleic acid.

For specific example, a procedure of purifying viral ribonucleic acid from new influenza A virus by using the automated purification and dispensation instrument and the nucleic purification kit of the apparatus for integrated real-time nucleic acid analysis is as follows.

Sample is collected from oral cavity and ansopharyngeal cavity by using a cotton swab. A tube for sample storage, in which virus transport media (VTm) is contained, is strongly shaken for 1 minute by using a vortex mixer, and thereby to enable virus to be effectively taken off from the cotton swab to the virus transport media, before the sample is used in purification of nucleic acid. The solution (PBS, normal saline, transport media, or the like) is well blended for 1 minute or longer by using the vortex mixer so that the virus can be moved from the cotton swab to the solution. 1 ml of a mixed sample solution is transferred into a 1.5ml test tube, followed by centrifugal separation at 13, 000 rpm for 5 to 10 seconds. 200 $\mu\ell$ of a supernatant is take off to be used in extraction of viral RNA.

The automated purification and dispensation instrument is switched on, and thus is initialized. The integrated real-time nucleic acid analysis apparatus manager software is largely divided into two, that is, a part of controlling the automated nucleic acid purification and dispensation instrument and a part of controlling the real-time nucleic acid amplifier. In order to extract virus RNA, a diagnosis kit to be tested is selected by clicking a picture of the automated purification and dispensation instrument, which is on the left side on a software main screen. When a diagnosis kit input box is clicked and a diagnosis kit for new influenza detection is selected, the kind of nucleic acid to be extracted is automatically selected. The kind of sample according to the type of patient sample is inputted on the input window of 'sample source type' . When the kind of the sample is inputted, the popup window for inquiring the state of the cartridge is automatically displayed. It is confirmed whether or not used wells are present, with reference to the buffer cartridge I, and when the used wells are present, the used wells are marked through clicking so that they are no longer used.

Residual wells of the cartridge I are automatically marked for the negative control and the positive control. A single NTC and a single PC are set as basis, and two NTPs and two PCs may be changeably set. If there is a list to be retested, the list to be retested is displayed in this step, and if number buttons for sample to be retested on the list are selected, wells are sequentially 'assigned'. A 'sample name' box is clicked, and sample information is inputted therein by using a bar code reader or a keyboard. In order to apply the inputted information to the apparatus for integrated real-time nucleic acid analysis, the 'apply' button is clicked.

20 $\mu\ell$ of internal positive control (IPC) is inputted in each of the wells on the first column and the second column of cartridge I of the nucleic acid purification kit, on the working plate. 20 $\mu\ell$ of a positive control (PC) is put into the well at a location of the positive control (PC), that is to say, at location A-1 of cartridge I, and 200$\mu\ell$ of tertiary distilled water (negative control, NTC) treated with DEPC is put into the well at location B-1 of cartridge I, correspondingly to the contents inputted in the apparatus for integrated real-time nucleic acid analysis . 200$\mu\ell$ of patient sample is put into each of the other wells at locations from C-1 to H-1 and A-2 to H-2. Here, nucleic acid samples surely need to be put in the cartridge correspondingly to locations inputted from the apparatus for integrated real-time nucleic acid analysis . Disposable filter tips and unit reaction tubes are loaded on racks within respective automated purification and dispensation instruments, correspondingly to locations of the wells of the cartridge, in which the standard material, the tertiary distilled water treated with DEPC, and the samples of patents are contained, respectively.

Also, the elution tube and the diagnosis kit for diagnosis test of new influenza, in which the purified nucleic acids are contained, are loaded on the freezing block. The prepared cartridge set and plastic consumable set are loaded in each of the automated purification and dispensation instruments. Then, the 'RUN' button on the integrated real-time nucleic acid analysis apparatus manager software is clicked to operate the automated purification and dispensation instruments, thereby purifying RNA of the new influenza virus from the samples of patients. When purification of nucleic acid is completed, the elution tube for nucleic acid storage and the diagnosis kit for diagnosis test are taken off from the freezing block. The elution tube for nucleic acid storage is capped by using a provided tube cap, and then stored at a temperature of -80°C. As for the diagnosis kit, an upper end of a strip tube is sealed by using an optical adhesive optical film, and then transferred to the nucleic acid amplifier. Then, real-time RT-PCR is performed on the diagnosis kit. When extraction is completed, 50uL of nucleic acid extraction solution is contained in each of the elution tube and the reaction tube for diagnosis kit within the nucleic acid purification and dispensation instrument.

In the present exemplary embodiment of the present invention, Exicycler™ 96 Real-Time Quantitative Thermal Block was adopted and used as the real-time nucleic acid amplifier, and AccuPowerDiagnostics Kit by Bioneer Company was adopted and used as the diagnosis kit.

The amplification results from the real-time nucleic acid amplifier are obtained by the following procedure. First, a figure of the real-time nucleic acid amplifier is clicked in the apparatus for integrated real-time nucleic acid analysis. Lists of samples of which extraction is completed are stored according to the kits, in the manager software. A corresponding list is selected by clicking the 'Get Sample List' button.

Here, the popup window for determining which location of the multi-well temperature circulation block the selected diagnosis kit is put is displayed. The samples are assigned to the multiple wells by selecting check boxes of the sample list according to the strips of the reaction tube. When each well is selected, the location of each diagnosis kits is determined. Here, the desired locations of the diagnosis kits are selected while a multi-well figure is displayed on the screen.

Considering the number of strip tubes of the diagnosis kit, a column number and a row number are selected to determine where the strip tubes are loaded in the temperature circulation block. For example, if the diagnosis kit in which the nucleic acids are dispensed is in an 8-strip type, the diagnosis kit is positioned at the center of the multi-well temperature circulation block of the nucleic acid amplifier by clicking the button for a corresponding location of 96 wells. When selection is completed, a box in which a data name is inputted is displayed. When a desired name is inputted and the button is clicked, a work list to be nucleic acid-amplified is generated.

The diagnosis kits are loaded on the multi-well temperature circulation block of the real-time nucleic acid amplifier correspondingly to the 'assigned' locations, and then the real-time nucleic acid amplifier software is run. When the operation of the real-time nucleic acid amplifier is completed, result data are transmitted to the manager software. In the apparatus for integrated real-time nucleic acid analysis, the 'update result' button is selected by clicking the 'tap' button on a menu part of the manager software of the controller, and thus, PCR results are brought. The results are brought by selecting the 'work list' button, which is generated on the left upper part of a newly opened window. The result values are confirmed in an excel file format in the order that the diagnosis kits are 'assigned' . Result graphs according to the respective samples can be analyzed in connection with an analysis program connected thereto. As for samples, of which results are confirmed, updating of the results is performed on the manager program. Otherwise, retrial from the amplification step, or retrial from the purification step is selected, and thus, a retrial list is created.

More specifically, respective reaction tube racks, a disposable tip rack, an elution tube rack, and a buffer cartridge I are loaded on a plate of the automated purification and dispensation instrument prepared within a biological safety cabinet (BSC) . Reaction tubes, disposable filter tips, elution tubes, and diagnosis kits are put in the racks respectively, correspondingly to the number of samples. Holes are formed in the sealing films of buffer cartridge I and buffer cartridge II by using a provided 'hole-punch', correspondingly to the number of samples and the number of positive controls (PCs) and negative controls (NTCs), which are to be used as standard samples . Referring to FIG. 5, 20$\mu\ell$ of plant internal positive control (IPC) is put into each well, correspondingly to the locations where the samples, the positive controls (PCs), and the negative controls (NTCs) are inputted. 200$\mu\ell$ of distilled water treated with DEPC is put in the each of the wells for the negative control (NTC) . 200$\mu\ell$ of positive control (PC) is added in each of the wells for the positive control (PC). 200$\mu\ell$ of samples of patient prepared above is put in each of the wells of the cartridge. After the wells are closed with transparent acrylic lids, the cartridges are put in the automated purification and dispensation instrument.

The automated purification and dispensation instrument is operated to bring a program for new influenza test. After confirmation of the instrument to be used, the wells, in which the positive control (PC) and the negative control (NTC) are respectively contained, are marked. With respect to the wells for samples except the wells for the positive control (PC) and the wells for the negative control (NTC), patient information (sex, age, part in charge, and the like) for the patient samples are inputted by a bar code reader or manually. The 'RUN' button is pressed to start extraction of Viral RNA.

After the extraction of Viral RNA is completed, when 'door' is opened and 'Base plate' is pulled, the elution tube rack, on which the elution tubes and the diagnosis kit are loaded, can be seen.

There is 50$\mu\ell$ of the extracted viral RNA in the elution tube and the diagnosis kit each. Therefore, there is no need of further addition of RNA or distilled treated with DEPC. The diagnosis kit necessary for new influenza test may be taken out from the elution tube rack, and directly applied in real time RT-PCR by using the real-time nucleic acid amplifier. Viral RNA contained in the elution tube is used at the time of retest. The extracted viral RNA is stored at a temperature of -80 °C until used, if possible. The elution tube rack is maintained in a temperature of 4°C in order to store the extracted nucleic acid.

A nucleic acid amplification analysis reagent contained in the diagnosis kit used in the present invention includes an analysis reagent containing all components necessary for amplification of target nucleic acid. The analysis reagent is constituted in a 'ready-to-use' type in order to real-time detect target DNA or RNA within the sample qualitatively or quantitatively, using the real-time nucleic acid amplifier.

The analysis reagent in the diagnosis kit used in the present invention includes buffer for reaction, $MgCl_2$, four kinds of dNTP, polymerase, primer, and detection label, which are necessary for amplification of nucleic acid, and selectively a stabilizer. The analysis reagent is made in a dry state. The detection label is characterized to be a fluorescent dye or a probe labeled with fluorescent dye. In addition, according to the present invention, a composition for the analysis reagent is preferably dried through freeze-drying, vacuum drying, heated drying, drying under reduced pressure, or the like.

As the diagnosis kit used in the present invention, for example, AccuPower PCR kit by Bioneer Company may be adopted and used. A primer, a dual-labeled fluorogenic probe, DNA polymerase, dNTPs, and a stabilizer, which are for amplifying a specific portion of a genome of New Influenza A(H1N1) and Influenza A virus each, are included in New Inf A(H1N1) & Inf A Premix by Bioneer Company. The amplification product is confirmed by measuring fluorescence bound to a specific probe during thermal cycling. Amplification according to cycling of PCR product is real-time measured by measuring a reporter dye taken off from the probe. Also, New Inf A H1N1 & Inf A positive controls are contained in the diagnosis kit for qualitative analysis of samples.

In the case where the nucleic acid amplification analysis reagent is used in an amplification step, the separated nucleic acid is put in the strip tube in which the analysis reagent is contained. Here, New Inf A H1N1 & Inf A positive control RNA reagent and internal positive control (IPC) RNA reagent are added in the tube. A mixture solution is prepared by mixing distilled water treated with DEPC and internal positive control (IPC) RNA in the calculated quantity, and 45$\mu\ell$ of the mixture solution is dispensed in each well of the strip.

The strip, in which dispensation of all reagents and RNA is completed, is closed with a lid or sealed with a sealing film, thereby fully mixing contents in the mixture solution. Here, a spin procedure is preferably performed by using Exispin™ (Cat.No: A-7040, Bioneer Co., KOREA), after the mixing. Then, the strip is loaded on the multi-well temperature circulation block of the real-time nucleic acid amplifier (Exicycler™ 96 Real-Time Quantitative Thermal Block, Bioneer Company), and then the real-time nucleic acid amplifier is run.

A main power switch, which is disposed at a lower part of a rear surface of the real-time nucleic acid amplifier (Exicycler™ 96), is turned on. When the 96-well multi-well temperature circulation block is moved in front of machine, PCR strips or plates are inputted according to the direction of the wells of the multi-well the temperature circulation block. When the 'door' button is again pressed, the 96-well multiwall temperature circulation block (thermal block) is moved inwardly. An operation program (Run ExiDiagnosis icon) of Exicycler™ 96 is run. In order to run an operation method necessary for PCR reaction, the "File-Design Experiment' button at the upper part is clicked.

Then, when the 'Select Diagnostics Kit window' is displayed, the 'New Inf A and InfA Real-Time PCR Kit' button is selected. When selection of 'Select Diagnostics Kit' is completed, an excel file, in which information on the position of strip within the 96-well multi-well temperature circulation block is inputted, is selected and opened.

When all selection is finished, a protocol and plate information display window, through which PCR reaction conditions suitable for kits and the selected positional information can be displayed, is confirmed.

When the 'Run tap' button is clicked, a new window, in which a file name for storing results can be inputted, is displayed. When information on experiment date and sample is inputted and the 'OK' button is pressed, PCR is started. Results can be confirmed from the analysis program by using the inputted file name.

The amplification results with respect to the amplification of nucleic acid are analyzed by using Exicycler™ 96 analysis program. 'PCR data file (.exe3)' to be analyzed is selected. Customized analysis is possible through 'Baseline subtraction (manual manner)' on "Flu. Graph' screen of the amplification result, and basic analysis results provided from the analysis program can be confirmed by using the 'auto scale' button. In the case of the negative control (NTC), New Inf A & Inf A RNA are not added. Therefore, FAM and Texasred fluorescent values are not measured. In the case of the internal positive control (IPC) RNA, TAMRA fluorescent values need to be measured throughout all the wells.

When PCR is performed, predetermined wells are assigned among wells, and for example, A1 and B1 are assigned for the negative controls (NTCs), and C1 and D1 are assigned for the positive controls (PCs). The positive control and the negative control are used as standard samples. If the two controls do not meet the reference, an error message may be generated in the analysis program. In the present invention, the software functions to 'Auto' analysis at the time of diagnostic analysis. The results are automatically analyzed, and the analyzed results are displayed.

**[Example 3]**

Example 3 is directed to simultaneous detection of internal positive controls and several samples used in separation and purification of nucleic acids, in a method for detecting target a nucleic acid using the apparatus for integrated real-time nucleic acid analysis as disclosed above.

(1) Design and application of plant internal positive control primer and probe

As for the plant internal positive control (IPC), a base sequence was selected between base sequence No. 4903 and No. 5709 (Movement protein gene, MP), and between base sequence No. 5712 and No. 6191 (Coat protein gene, CP) of a genome RNA (GeneBank. ACCESSION No. NC001367) of a tobacco mosaic virus (TMV) such that it had a length of 18 to 28 bp and a Tm value of 55°C to 62°C, and thus, the selected base sequence was used as a forward primer and a reward primer.

In addition, any base sequence was selected from the base sequences such that it had a length of 19 to 30 bp and a Tm value of 67°C to 72°C, and the selected base sequence was used as the prober [table 1]. Tm values are checked by using Primer3 Plus program.

[table 1]

| No. | | Forward Primer | Reward Primer | TaqMan Probe (Forward) |
|---|---|---|---|---|
| 1 | MP1 | AGATTTCAGTTCAAGGTCGTTC | GAAACCCGCTGACATCIT | ACGCGATGAAAAACGTCTGGCAAGT |
| 2 | MP2 | TCAGTTCAAGGTCGTTCC | GAGAAAGCGGACAGAAACC | ACGCGATGAAAAACGTCTGGCAAGT |
| 3 | MP3 | ACAATTGCAGAGGAGGTG | TGGGAACGACCTTGAACT | CTGGTGGACAAAAGGATGGAAAGAGC |
| 4 | CP1 | AAGCTCGAACTGTCGTTC | CTAACAGTGCTGTGACTA | AGACAATTCAGTGAGGTGTGGAAACCTT |
| 5 | CP2 | GGTGTGGAAACCTTCAC | CAACTTCTATTATTCTATTTCTAGTGTC | AGTGACTTTAAGGTGTACAGGTACAATGC |
| 6 | CP3 | TGACTTTAAGGTGTACAGG | CGTCTACTCTACGAGTAG | ATAGAAGTTGAAAATCAGGCGAACOOC |

A procedure for finding out appropriate primer combinations was performed by combining primers of tobacco mosaic virus (TMV) according to the sets and using SYBR Grenn (nucleic acid reagent for detection of nucleic acid) and Exicycler™ Quantitative Thermal Block (Bioneer Company, Korea) as a real-time quantitative amplifier.

Six combined sets of tobacco mosaic virus (TMV) primer, a reagent for dying nucleic acid, distilled water (D.W.), and a tobacco mosaic virus template, were added and then subjected to real-time RT-PCR. These were subjected to 45 cycles of 95°C for 10 minutes for denaturation, 95°C for 20 seconds, and 55°C for 30 seconds. As respective PCR cycles were performed, the amplified fluorescent values were consecutively measured once every cycle after reaction at 55°C for 30 seconds every cycle.

As a result, Primer sets 1, 2, and 3 (MP sets 1, 2, and 3) have a higher PCR rate than Primer sets 4, 5, and 6 (CP sets 1, 2, and 3), and thus, MP sets 1, 2, and 3 were selected.

In an experiment thereafter, an appropriate probe set selection procedure was further performed by using a probe detecting a nucleic acid of specific sequence instead of the unspecific reagent for dying nucleic acid. 10×Buffer 5$\mu\ell$, MMLV 600U, Taq 7.5 unit, dNTP 20mM 3$\mu\ell$, stabilizer, a primer of the optimized concentration (15p), a probe, and the like, and tobacco mosaic virus (TMV) RNA extracted from diseased leaves added thereto, reach total volume of 50$\mu\ell$ within one tube, and real-time RT-PCR was performed. This resulting material was subjected to RT-PCR at 45°C for 15 minutes, followed by 45 cycles of 95°C for 10 minutes for denaturation, 95°C for 20 seconds, and 55°C for 30 seconds.

Among the tobacco mosaic virus primer and probe sets 1, 2, and 3, Probe set 1 (MP 1) had the best PCR amplification efficiency, and Probe set 3 (MP 3) and Probe set 2 (MP 2) had followed Probe set 1 (MP 1) in that order.

Standard template real-time RT-PCR application experiment was performed by using Probe sets 1 and 3 of the probe sets, using tobacco mosaic virus (TMV) RNA as the internal positive control RNA in the RT-PCR mixture solution having the above composition, and adding new influenza RNA as a template . Results of the experiment confirmed that, even though Probe Sets 1 (MP1) and 3 (MP3) reacted with $1\times10^6$ copy internal positive control RNA, there was no large effect on amplification of the new influenza RNA template and amplification of the internal positive controls was well made in an independent manner.

(2) Preparation of plant internal positive control, Application thereof during a nucleic acid extraction procedure, and Application of real-time RT-PCR

In order to prepare a plant internal positive control usable with biological samples, first, tobacco mosaic virus was grown. 100 to 200 tobacco *Nicotiana tabaccum*cv. *Samsun* seeds (available from a plant virus laboratory of Rural Development Administration) were sown, and 10 days later, buds spouted. After 10 days from then, each object was transplanted to a small flowerpot, and then was further grown for 10 days Tobacco mosaic virus disease leaves (available from a plant virus laboratory of Rural Development Administration) were put in a mortar, and then ground with 0.01 M Phosphate Buffer(pH 7.2), thereby preparing a juice. The prepared juice was covered on the tobacco leaves on which carborundom was sprinkled, and thus, inoculation of TMV was conducted through injury of the leaves.

After 10 days of inoculation, mosaic spots, leaf distortion, and the like were observed on the tobacco leaves. The reproductive yield of tobacco mosaic virus was increased by further growing the tobacco leaves for about 20 days. Then, 30g of TMV disease leaves were harvested, and experiments for purifying virus particles were performed.

Specifically, 30g of tobacco mosaic virus (TMV) disease leaves, 90 ml of 0.1M Phosphate Buffer (pH7.2), and 0.6 ml of β-mecaptoethanol were put in a mixer, followed by grinding of the leaves and filtering with gauze, and then 12 ml of n-butanol was added thereto, followed by stirring for 1 hour 30 minutes at 4°C. The resultant mixture was subjected to centrifugal separation of 8000 rpm at 4°C for 20 minutes, and then only the supernatant was collected, and then filtered with Mira fiber cloth. PEG having 8% of the volume of the supernatant and 0.1M NaCl were added thereto, followed by stirring for 3 hours (maintained at 4°C).

The mixture thus obtained was subjected to centrifugal separation of 10000 rpm at 4°C for 20 minutes, and then the supernatant was removed and pallet was well dissolved in 7.5 ml of 0.1M Phosphate buffer. The resulting material was again subjected to centrifugal separation of 10000 rpm at 4°C for 20 minutes, and then the supernatant was taken out and subjected to centrifugal separation of 28000 rpm at 4°C for 2 hours 30 minutes. After removal of the supernatant, the residual pallet is dissolved in distilled water in which 300$\mu\ell$ of DEPC was added, thereby extracting tobacco mosaic virus (TMV) particles. The extracted particles were confirmed by SDS-PAGE and electron microscope images. The concentration of virus was quantitated by measuring absorbance (260nm) with a UV spectrometer (Shimazu Company product, Japan) and using the following equation.

$$[\text{absorbance at 260nm}/\ 3.09\ (\text{TMV absorbance coefficient})]$$

$$\times \text{ dilution factor}$$

Based on the concentration quantitation result, RNA copy number was calculated by the following equation.

$$6.02\times10^{23}\times\text{concentration (concentration measured by a UV spectrometer, mg/ml) / 6395 (size of tobacco mosaic virus genome, bp)}\times 330$$

An optimal concentration selection experiment was performed in order to apply the tobacco mosaic virus particles extracted from the above-mentioned experiment from the purification step, which is an RNA extraction step. First, 200μℓ of a dilution solution of tobacco mosaic virus particles was used to extract 50μℓ through a nucleic acid separation and purification instrument (ExiPrep™, Bioneer Company product, Korea), and this was used as internal positive control RNA. 1μℓ of the internal positive control RNA was put into a tube in which 10×Buffer 5μℓ, MMLV 600U, Taq 7. 5 unit, dNTP 20mM 3μℓ, stabilizer, a primer of the optimized concentration (15p), a probe, and the like, and new influenza RNA as a standard template were added, and distilled water was added thereto, thereby reaching total volume of 50μℓ. Then, real-time RT-PCR was performed in the nucleic acid amplifier. This resulting material was subjected to RT-PCR at 45°C for 15 minutes, followed by 45 cycles of 95°C for 10 minutes for denaturation, 95°C for 20 seconds, and 55°C for 30 seconds.

As the result, it was firmed that $2\times10^8$ to $2\times10^9$ /20μℓ of the internal positive control RNA per one reaction solution exhibited the optimum PCR efficiency at the time of separation and purification.

Next, the tobacco mosaic particles extracted together with the new influenza samples are applied in experiments from the RNA extraction step to real-time RT-PCR, and then compared with the existing used internal positive control (internal positive control contained in AccuPower Diagnostics kit of Bioneer Company (mouse DVL gene plasmid)). At the time of RNA extraction, $2\times10^8$ to $2\times10^9$ copy/20μℓ of RNA in conformity to concentrations selected by the above experiments were mixed with 200 μℓ of New Influenza sample, and they are separated and extracted by the nucleic acid purification and dispensation instrument. At the time of real-time RT-PCR in the nucleic acid amplifier, the primer and probe sets 1 and 3 (MP1 and MP3) selected in Example 2 were used.

The experiment result confirmed that the optimum concentration of the tobacco mosaic virus particles at the time of RNA extraction is $2\times10^8$copy/20μℓ. Therefore, it is preferable to mix 20μℓ of $1\times10^7$ copy/μℓ of tobacco mosaic virus particle with 200 μℓ of sample in the purification step. In addition, it was shown that Primer and Probe set 1 (MP 1) has higher efficiency in amplification of the TMV internal positive control.

In addition, when this result was compared with the existing internal positive control reaction results with respect to the same new influenza sample, it was confirmed that, even though the tobacco mosaic virus was extracted together with the sample and they are subjected to reaction, there was no large effect on amplification of the new influenza RNA template and amplification of the plant internal positive control was well made in an independent manner, and the result was good. Further, it was confirmed that fluorescent value was higher, as compared with the internal positive control used in the existing diagnosis kit by Bioneer Company, and thus reaction intensity was higher.

In addition, in order to confirm that it is possible to perform quantitative analysis, RNA extraction was performed by the nucleic acid separation and purification instrument and real-time RT-PCR was performed in the nucleic acid amplifier while the tobacco mosaic virus particles were used in contents of $10^2$ to $10^8$ copy/reaction.

As the result, the TMV particles applied in concentrations of $10^2$ to $10^8$ copy/reaction are detectable at least up to $10^3$ copy/reaction (FIG. 6). When a standard graph of standard template real-time RT-PCR was made, a slope is -0.28 and $R^2$ value was 1 (FIG. 6). Here, $R^2$ represents a correlation coefficient showing the linearity of the graph in the standard graph of the real-time PCR, and it means that as the $R^2$ value is closer to 1 (as the graph is closer to the straight line), PCR is more preferably performed. As compared with the standard graph when the standard template real-time RT-PCR was performed on TMV RNA, the concentration of the tobacco mosaic virus particles has a tendency to be reduced to 1/10 after RNA extraction. However, the standard graph showed a uniform space according to the concentrations of tobacco mosaic virus, which confirmed that quantitative analysis, was possible.

(3) Real-time PCR using different four kinds of nucleic acids (DNA/RNA)

It was confirmed whether it is possible to detect and diagnosis different kinds of target nucleic acids separated and purified from various biological samples. As a nucleic acid amplifier constituting an apparatus for integrated real-time nucleic acid analysis of the as disclosed above, Exicycler™ Quantitative Thermal Block (Bioneer Company product, Korea) was used and diagnosis kits by Bioneer Company were used. As the diagnosis kits used in the real-time PCR, four kinds of diagnosis kits, that is, HBV Quantitative PCR Kit (Bioneer Company product, Cat. No. HBV-1111), HCV Quantitative RT-PCR Kit (Bioneer Company product, Cat. No. HCV-1111), New InfA (H1N1) Real-Time RT-PCR Kit (Bioneer Company product, Cat. No. SIV-1111), and CT&NG Real-Time PCR Kit (Bioneer Company product, Cat. No. STD2A-1111) were used.

The HBV Quantitative PCR Kit is constituted to enable quantitative analysis by amplifying HBV DNA extracted from the serum sample, in order to determine whether hepatitis B infection occurs or not. As for the HBV Quantitative PCR Kit, a PCR mixture solution of 10×PCR Buffer 5 $\mu\ell$, wTfi polymerase 5U, dNTP 20 mM 5 $\mu\ell$, thermostable pyrophosphatase, PPi, stabilizer, and the like, and a primer and a probe designed to specifically amplify only HBV DNA are freeze-dried (Table 2).

The CT&NG Real-Time PCR Kit is constituted to simultaneously amplify Chlamydia Trachomatis (CT) DNA and Neisseria gonorrheae (NG) DNA extracted from Urine or Swab samples in one reaction tube, in order to determine whether venereal infection occurs or not. The CT&NG Real-Time PCR Kit has the same PCR mixture solution as the HBV diagnosis kit, and a primer and a probe designed to specifically amplify only CT DNA or NG DNA are freeze-dried (Table 2).

The two above diagnosis kits have similarity in amplification of DNA, but have a difference in that the HBV diagnosis kit amplifies a single kind of viral DNA extracted from the serum and the CT&NG diagnosis kit simultaneously amplifies two kinds of bacterial DNA, that is to say, CT DNA and NG DNA extracted from the urine at one time. In addition, the HBV diagnosis kit is designed for quantitative analysis of DNA and the CT&NG diagnosis kit is designed for qualitative analysis of DNA.

[Table 2]

| | Forward Primer | Reward Primer | Probe |
|---|---|---|---|
| HBV | CCAATCACTCACCAACCTCTTGT | AGCAGGATGAAGAGGAATATGATAAAA | TCCTGGCTATCGCRGGATGTGTTCTGC |
| HCV | GCGGAACCGGTGAGTACAC | TCAGGCAGTACCACaaGGC | CGTGCCCCCGCAAGACTGCT |
| CT | GGTATTAGTATTTGCCGCTTTGAGT | GTCGATCATAAGGCTTGGTTCAG | CTGCTTCCTCCTTGCAAGCTCTGCC. |
| NG | CGTAATACCGCATACGTCTTGAGA | CGCCAACCAGCTAATCAGATATC | CTTCGGGCCTTGCGCTATCCGA |
| New H1N1 | GGCTGGATCCTGGGAAATC | TCGATGAAATCTCCTGGGTAAC | ACTCTCCACAGCAAGCTCATGGTCC |

The HCV Quantitative RT-PCR Kit, which is developed in order to diagnose whether hepatitis C infection occurs or not, is similar to the HBV diagnosis kit in that quantitative analysis is possible by amplifying nucleic acid extracted from the serum, but different from the HBV diagnosis kit in that HBV diagnosis kit amplifies viral DNA and the HCV diagnosis kit amplifies viral RNA through RT-PCR.

The New InfA (H1N1) Real-Time RT-PCR Kit, which was recently developed in order to diagnose whether new influenza infection occurs or not, has a similarity in that target RNA is amplified, but has a difference in that RNA is extracted from an respiratory organ instead of serum, as compared with the HCV diagnosis kit. In addition, the New InfA (H1N1) Real-Time RT-PCR Kit is designed for qualitative analysis, unlike the HCV diagnosis kit allowing quantitative analysis. As for the HCV diagnosis kit or New InfA(H1N1) diagnosis kit for RT-PCR of RNA, an RT-PCR mixture solution of 10× RT Buffer 5 $\mu\ell$, MMLV 600U, wTfi 5 unit, dNTP 20 mM 3 $\mu\ell$, DTT 50 mM, RNasin 15U, stabilizer, and the like, and a primer and a probe designed to selectively amplify HCV RNA or New InfA (H1N1) RNA are freeze-dried (Table 2). The primer and the probe included in each diagnosis kit are designed to have similar Tm values ranging from 55°C to 57°C, and thus, it is possible to perform PCRs using four different kinds of diagnosis kits at the same time.

In order to simultaneously perform PCRs or RT-PCRs using four different kinds of diagnosis kits in the nucleic acid amplifier, first, 5$\mu\ell$ of template DNA or RNA, 1$\mu\ell$ of DNA or RNA for internal positive control (IPC), and 44$\mu\ell$ of distilled water are put into the dried PCR mixture, and mixed to reach the total volume of 50$\mu\ell$. The template RNA or DNA was synthesized by using a specific portion of each target, which is selected through an alignment procedure, by a gene synthesis method (NBiochem. Biophys. Res. Commun.1998, 248, 200-203) in Bionner Company, and then cloned by using pGEM-T-Easy Vector (Promega Company, USA).

The HBV, HCV, CT&NG, and New InfA(H1N1) diagnosis kits were loaded on the temperature circulation block of a single real-time nucleic acid amplifier, and columns and wells are set on the software program correspondingly to the assigned area. PCRs were simultaneously performed under the PCR condition of a reverse transcription reaction at 45 °c for 15 minutes, followed by 45 cycles of 95°C for 5 minutes for denaturation, 95°C for 5 seconds, and 55°C for 5 seconds. As for the HBV diagnosis kit and the HCV diagnosis kit, $10^1$ to $10^7$ copy/reaction of template DNAs were respectively used for quantitative analysis. As for the CT&NG diagnosis kit and the New Inf A(H1N1) diagnosis kit, only one kind of concentration of positive control template DNA or RNA was used for qualitative analysis.

The DNA or RNA for the internal positive control is a gene designed to be independently amplified without having no effects on amplification of template DNA or RNA, and used to verify that there is no problems in PCR, by confirming whether the internal positive control DNA or RNA is appropriately amplified or not, from the result of the negative control in which the template DNA or RNA is never amplified.

As the result, the template DNA of the HBV diagnosis kit applied in concentrations of $10^1$ to $10^7$ copy/reaction are detectable at least up to $10^1$ copy/reaction (FIG. 8), and when a standard graph of standard template real-time RT-PCR was made, a slope was -0.28 and $R^2$ value was 0.9997 (FIG. 9). Here, $R^2$ represents a correlation coefficient showing the linearity of the graph in the standard graph of the real-time PCR, and it means that as the $R^2$ value is closer to 1 (as the graph is closer to the straight line), PCR is more preferably performed. Also, in the case of the HCV diagnosis kit, the template RNA was detectable at least up to $10^1$ copy/reaction (FIG. 10), and when a standard graph of standard template real-time RT-PCR was made, a slope was -0.29 and $R^2$ value was 0.9998 (FIG. 11). The above results confirmed that PCRs of RNA and DNA could be simultaneously performed in a single real-time nucleic acid amplifier, regardless of a difference between RNA and DNA.

Also, in the case of the CT&NG diagnosis kit designed for qualitative analysis, when PCRs were performed together with the HBV and HCV diagnosis kits, which are quantitative analysis kits, at the same time, all of CT positive control DNA, NG positive control DNA, and DNA for the internal positive control are normally amplified. In order to confirm the detectable limit, $10^1$ to $10^7$ copy/reaction of CT and NG template DNAs were subjected to reaction. The results confirmed that $10^1$ copy/reaction of CT and NG template DNAs all were detectable. When a standard graph of standard template real-time RT-PCR was made, a slope was -0.28 and $R^2$ value was 0.9997 in CT and a slope was -0.30 and $R^2$ value was 0.9996 in NG.

Also, in the case where the New Inf A(H1N1) diagnosis kit was applied in the similar method to the CT&NG diagnosis kit, it was confirmed that the positive control RNA was normally amplified. In order to confirm the detectable limit, $10^1$ to $10^7$ copy/reaction of new Inf A (H1N1) template DNAs were subjected to reaction. The results confirmed that $10^1$ copy/reaction of the new Inf A (H1N1) template DNA was detectable. When a standard graph of standard template real-time RT-PCR was made, a slope was -0.28 and $R^2$ value was 0.9996. Therefore, it was confirmed that PCRs using even kits designed for quantitative analysis or qualitative analysis can be simultaneously performed.

The above experiment results confirmed that, in performing PCR and RT-PCR by using a single real-time nucleic acid amplifier included in the apparatus for integrated real-time nucleic acid analysis, DNA and RNA extracted from different samples, such as serum, urine, a respiratory organ, and the like can be simultaneously amplified under the same condition even when four different kinds of diagnosis kits designed for quantitative analysis or qualitative analysis are used.

## Claims

**1.** A method for simultaneously detecting a plurality of target nucleic acids performed by an integrated real-time nucleic acid analysis system comprising a plurality of automated separation and purification instruments, a real-time nucleic acid amplifier, and a controller, the method comprising:

1) separating nucleic acids from various biological samples and purifying the separated nucleic acids, by the plurality of automated separation and purification instruments;
2) applying the purified nucleic acids to a plurality of reaction tubes, wherein the plurality of reaction tubes includes a first reaction tube for detecting a first target nucleic acid and a second reaction tube for detecting a second target nucleic acid different from the first target nucleic acid;
3) assigning wells of a multi-well temperature circulation block to the plurality of reaction tubes according to the target nucleic acid, wherein a first well is assigned to the first reaction tube and a second well is assigned to the second reaction tube, and storing information on the samples for each well of each of the group of wells;
4) loading each of the plurality of reaction tubes prepared in step 2) to a well corresponding to the information on the biological samples, wherein the first reaction tube is loaded to the first well and the second reaction tube is loaded to the second well; and
5) simultaneously amplifying the first target nucleic acid loaded on the first well and the second target nucleic acid loaded on the second well under a same thermal condition and performing at least one of qualitative analysis and quantitative analysis on the respective target nucleic acids separated from the plurality of biological samples, thereby to obtain amplification results, wherein a first primer set and a first probe is added to the first reaction tube for amplifying and detecting the first target nucleic acid, and a second primer set and a second probe is added to the second reaction tube for amplifying and detecting the second target nucleic acid, **characterized in that**:

- in step 1) nucleic acids are also separated from standard samples;
- in step 3) a first information on a standard sample and biological sample of the first reaction tube and a second information on a standard sample and biological sample of the second reaction tube is stored; and

wherein the first and second primer sets and the first and second probes are different from each other and have a similar melting point such that simultaneously amplifying and analyzing the nucleic acids are performed under the same thermal conditions, and
wherein the first target nucleic acid is separated from a first biological sample and the second target nucleic acid is separated from a second biological sample different from the first biological sample.

**2.** The method of claim 1, wherein in the step 2), the reaction tube is prepared by applying the separated and purified nucleic acid solution to a reaction tube, which contains dried component necessary for amplification of nucleic acid and the separated and purified solution and the dried components are mixed in the reaction tube.

## Patentansprüche

**1.** Verfahren zum gleichzeitigen Nachweisen einer Vielzahl von Target-Nucleinsäuren, das durch ein integriertes Echtzeit-Nucleinsäure-Analysesystem, umfassend eine Vielzahl von automatisierten Trennungs- und Reinigungsgeräten, einen Echtzeit-Nucleinsäure-Amplifier und einen Kontroller, durchgeführt wird, wobei das Verfahren umfasst:

1) Trennen von Nucleinsäuren aus verschiedenen biologischen Proben und Reinigen der getrennten Nucleinsäuren durch die Vielzahl von automatisierten Trennungs- und Reinigungsgeräten;
2) Aufbringen der Nucleinsäuren in eine Vielzahl von Reaktionsröhrchen, wobei die Vielzahl von Reaktionsröhrchen ein erstes Reaktionsröhrchen zum Nachweisen einer ersten Target-Nucleinsäure und ein zweites Reaktionsröhrchen zum Nachweisen einer zweiten Target-Nucleinsäure, die sich von der ersten Target-Nucleinsäure unterscheidet, umfasst;
3) Zuweisen von Vertiefungen eines Temperatur-Zirkulationsblocks mit mehreren Vertiefungen der Vielzahl von Reaktionsröhrchen entsprechend der Target-Nucleinsäure, wobei eine erste Vertiefung dem ersten Reaktionsröhrchen zugewiesen wird und eine zweite Vertiefung dem zweiten Reaktionsröhrchen zugewiesen wird, und Speichern von Information über die Proben für jede Vertiefung jeder Gruppe von Vertiefungen;
4) Laden jedes der Vielzahl von Reaktionsröhrchen, die in Schritt 2 hergestellt wurden in eine Vertiefung entsprechend der Information über die biologischen Proben, wobei das erste Reaktionsröhrchen in die erste Vertiefung geladen wird und das zweite Reaktionsröhrchen in die zweite Vertiefung geladen wird; und
5) gleichzeitiges Amplifizieren der ersten Target-Nucleinsäure, die in die erste Vertiefung geladen ist, und der zweiten Target-Nucleinsäure, die in die zweite Vertiefung geladen ist, unter einer gleichen thermischen Bedingung, und Durchführen wenigstens einer qualitativen Analyse und quantitativen Analyse an den jeweiligen Target-Nucleinsäuren, die aus der Vielzahl biologischer Proben getrennt wurden, wodurch Amplifikationsergebnisse erhalten werden, wobei ein erster Primersatz und eine erste Sonde zum ersten Reaktionsröhrchen zum Amplifizieren und Nachweisen der ersten Target-Nucleinsäure zugegeben wird, und ein zweiter Primersatz und eine zweite Sonde zum zweiten Reaktionsröhrchen zum Amplifizieren und Nachweisen der zweiten Target-Nucleinsäure zugegeben wird, **dadurch gekennzeichnet, dass**:

- in Schritt 1) Nucleinsäuren auch aus Standardproben getrennt werden;
- in Schritt 3) eine erste Information über eine Standardprobe und eine biologische Probe des ersten Reaktionsröhrchens und eine zweite Information über eine Standardprobe und eine biologische Probe des zweiten Reaktionsröhrchens gespeichert wird; und

wobei der erste und zweite Primersatz und die erste und zweite Sonde unterschiedlich voneinander sind und einen ähnlichen Schmelzpunkt haben, sodass gleichzeitiges Amplifizieren und Analysieren der Nucleinsäuren unter den gleichen thermischen Bedingungen durchgeführt werden, und
wobei die erste Target-Nucleinsäure aus einer ersten biologischen Probe getrennt wird und die zweite Target-Nucleinsäure aus einer zweiten biologischen Probe, die sich von der ersten biologischen Probe unterscheidet, getrennt wird.

**2.** Verfahren gemäß Anspruch 1, wobei im Schritt 2) das Reaktionsröhrchen durch Aufbringen der getrennten und gereinigten Nucleinsäurelösung in ein Reaktionsröhrchen, das eine getrocknete Komponente enthält, die für die Amplifikation von Nucleinsäure notwendig ist, vorbereitet wird, und die getrennte und gereinigte Lösung und die getrockneten Komponenten in das Reaktionsröhrchen gemischt werden.

## Revendications

**1.** Procédé pour détecter simultanément une pluralité d'acides nucléiques cibles mis en œuvre par un système d'analyse intégrée d'acides nucléiques en temps réel comprenant une pluralité d'instruments automatisés de séparation et de purification, un amplificateur d'acide nucléique en temps réel, et un dispositif de commande, le procédé comprenant :

1) la séparation d'acides nucléiques à partir de divers échantillons biologiques et la purification des acides nucléiques séparés, par la pluralité d'instruments automatisés de séparation et de purification ;
2) l'application des acides nucléiques purifiés à une pluralité de tubes de réaction, dans lequel la pluralité de tubes de réaction inclut un premier tube de réaction pour détecter un premier acide nucléique cible et un second tube de réaction pour détecter un second acide nucléique cible différent du premier acide nucléique cible ;
3) l'attribution de puits d'un bloc multi-puits à circulation de température à la pluralité de tubes de réaction en fonction de l'acide nucléique cible, dans lequel un premier puits est attribué au premier tube de réaction et un second puits est attribué au second tube de réaction, et le stockage d'informations sur les échantillons pour chaque puits de chacun du groupe de puits ;
4) le chargement de chacun de la pluralité de tubes de réaction préparés dans l'étape 2) dans un puits correspondant aux informations sur les échantillons biologiques, dans lequel le premier tube de réaction est chargé dans le premier puits et le second tube de réaction est chargé dans le second puits ; et
5) simultanément l'amplification du premier acide nucléique cible chargé sur le premier puits et du second acide nucléique cible chargé sur le second puits dans une même condition thermique et la mise en œuvre d'au moins l'une parmi une analyse qualitative et une analyse quantitative sur les acides nucléiques cibles respectifs séparés à partir de la pluralité d'échantillons biologiques, pour ainsi obtenir des résultats d'amplification, dans lequel un premier jeu d'amorces et une première sonde sont ajoutés au premier tube de réaction pour amplifier et détecter le premier acide nucléique cible, et un second jeu d'amorces et une seconde sonde sont ajoutés au second tube de réaction pour amplifier et détecter le second acide nucléique cible, **caractérisé en ce que** :

- dans l'étape 1) des acides nucléiques sont également séparés à partir d'échantillons étalons ;
- dans l'étape 3) une première information sur un échantillon étalon et un échantillon biologique du premier tube de réaction et une seconde information sur un échantillon étalon et un échantillon biologique du second tube de réaction sont stockées ; et

dans lequel les premier et second jeux d'amorces et les première et seconde sondes sont différents les uns des autres et présentent un point de fusion similaire de sorte que simultanément l'amplification et l'analyse des acides nucléiques sont effectuées dans les mêmes conditions thermiques, et
dans lequel le premier acide nucléique cible est séparé à partir d'un premier échantillon biologique et le second acide nucléique cible est séparé d'un second échantillon biologique différent du premier échantillon biologique.

**2.** Procédé selon la revendication 1, dans lequel dans l'étape 2), le tube de réaction est préparé par l'application de la solution d'acide nucléique séparée et purifiée à un tube de réaction, qui contient un composant sec nécessaire pour l'amplification d'un acide nucléique et la solution séparée et purifiée et les composants secs sont mélangés dans le tube de réaction

【FIG. 1】

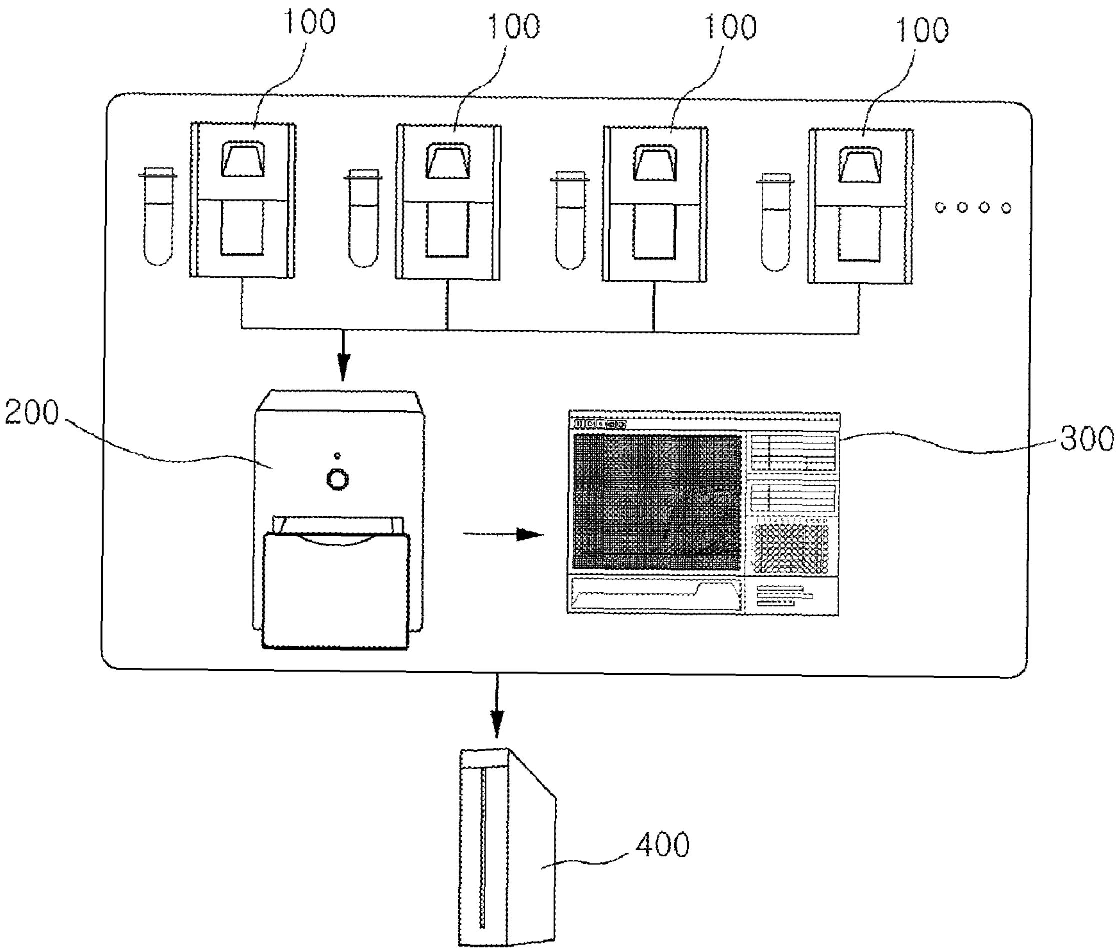

【 FIG. 2】

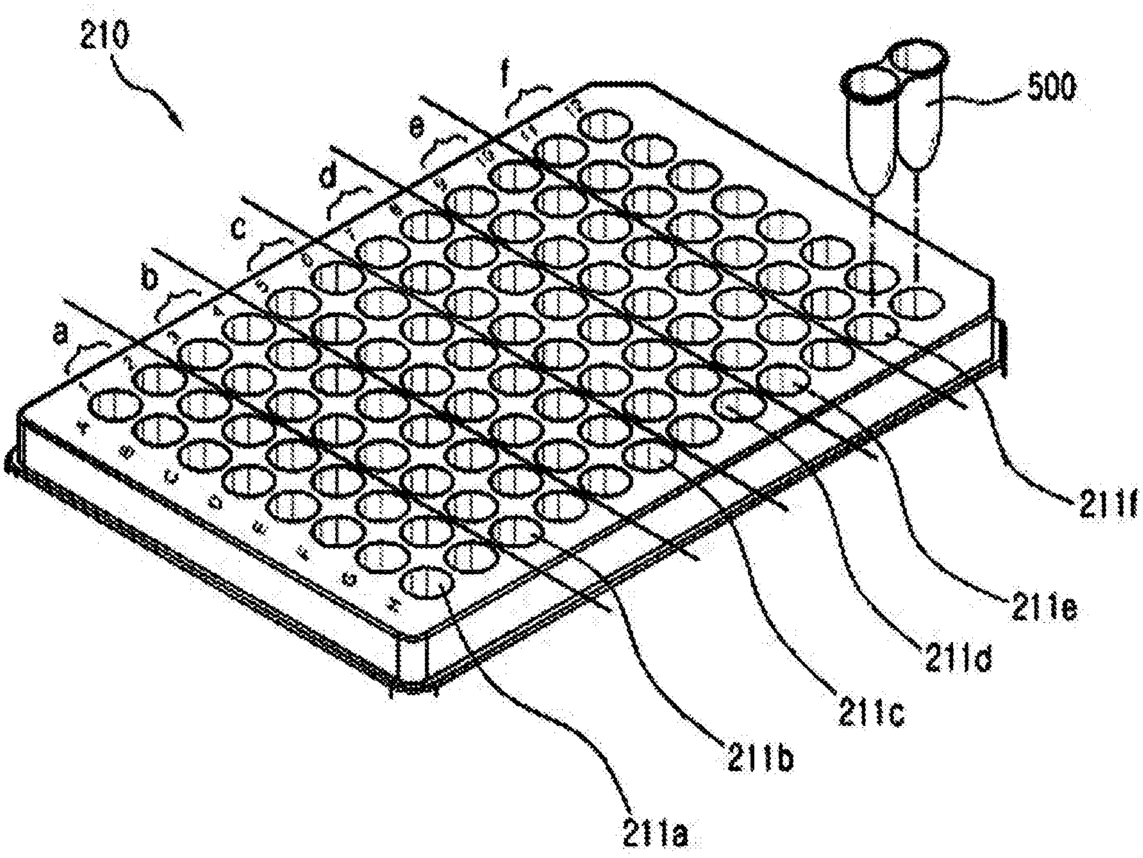

【 FIG. 3】

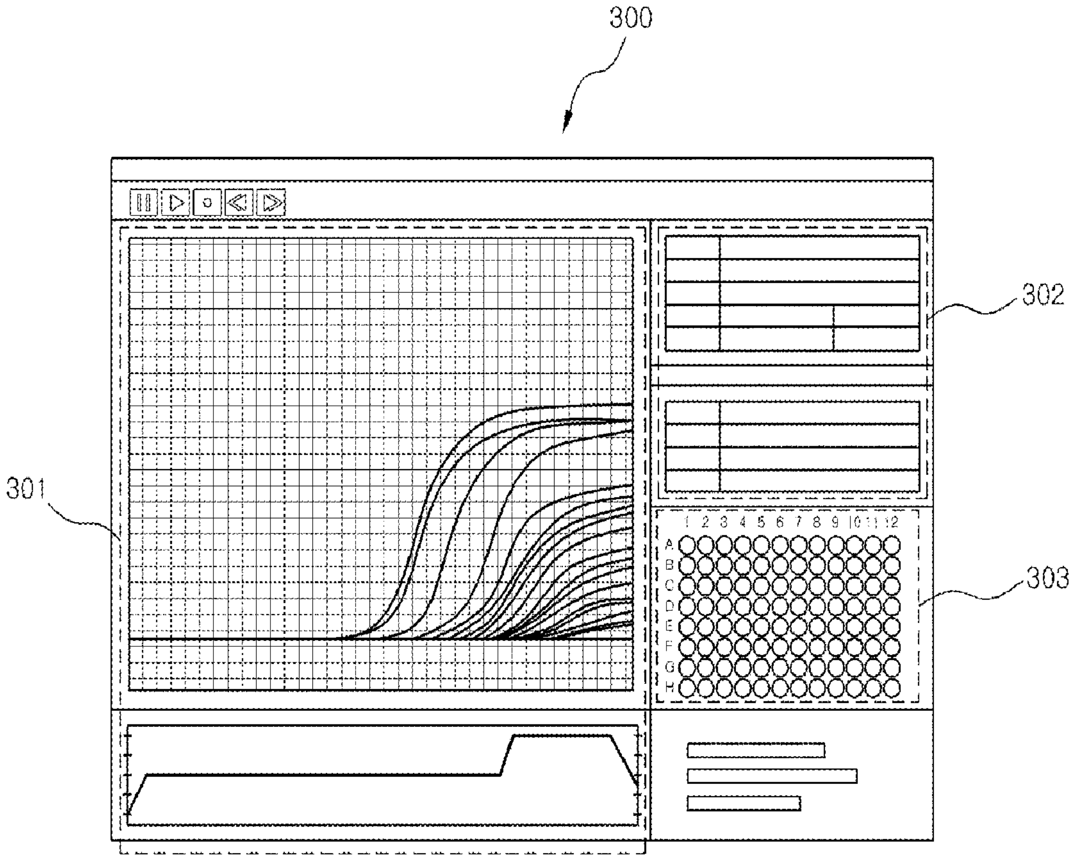

【 FIG. 4】

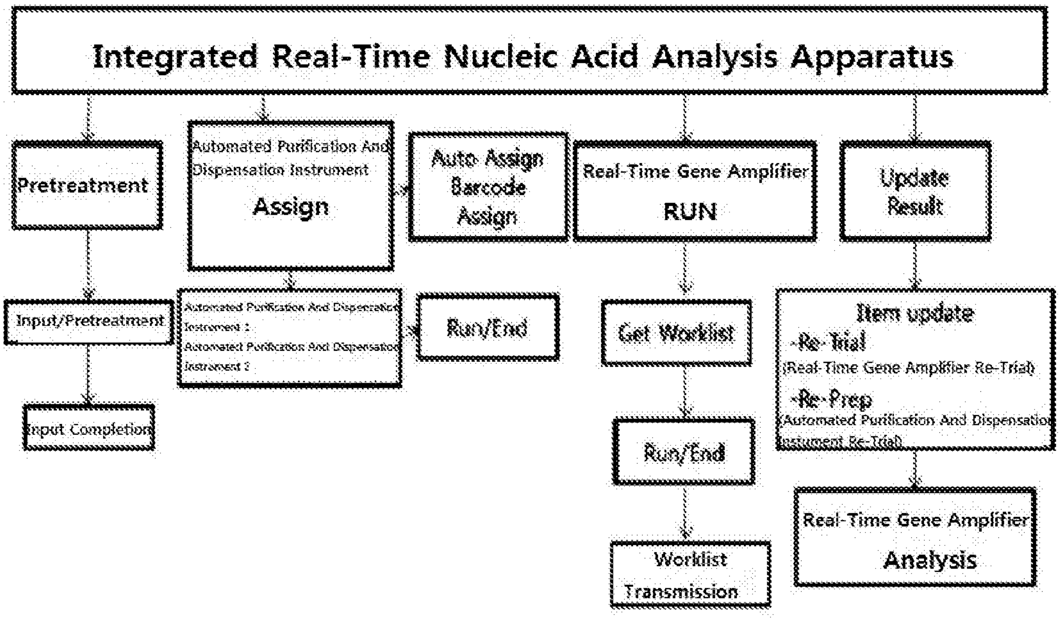
Integrated Real-Time Nucleic Acid Analysis Apparatus
Pretreatment
Automated Purification And Dispensation Instrument
Assign
Auto Assign Barcode Assign
Real-Time Gene Amplifier
RUN
Update Result
Input/Pretreatment
Automated Purification And Dispensation Instrument 1
Automated Purification And Dispensation Instrument 2
Run/End
Get Worklist
Item update
-Re-Trial
(Real-Time Gene Amplifier Re-Trial)
-Re-Prep
(Automated Purification And Dispensation Instument Re-Trial)
Input Completion
Run/End
Real-Time Gene Amplifier
Analysis
Worklist Transmission

【 FIG. 5】

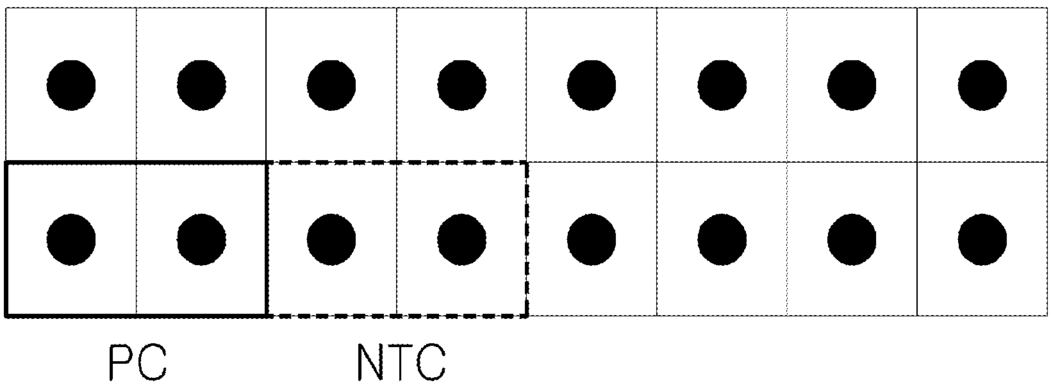
PC
NTC

【 FIG. 6】

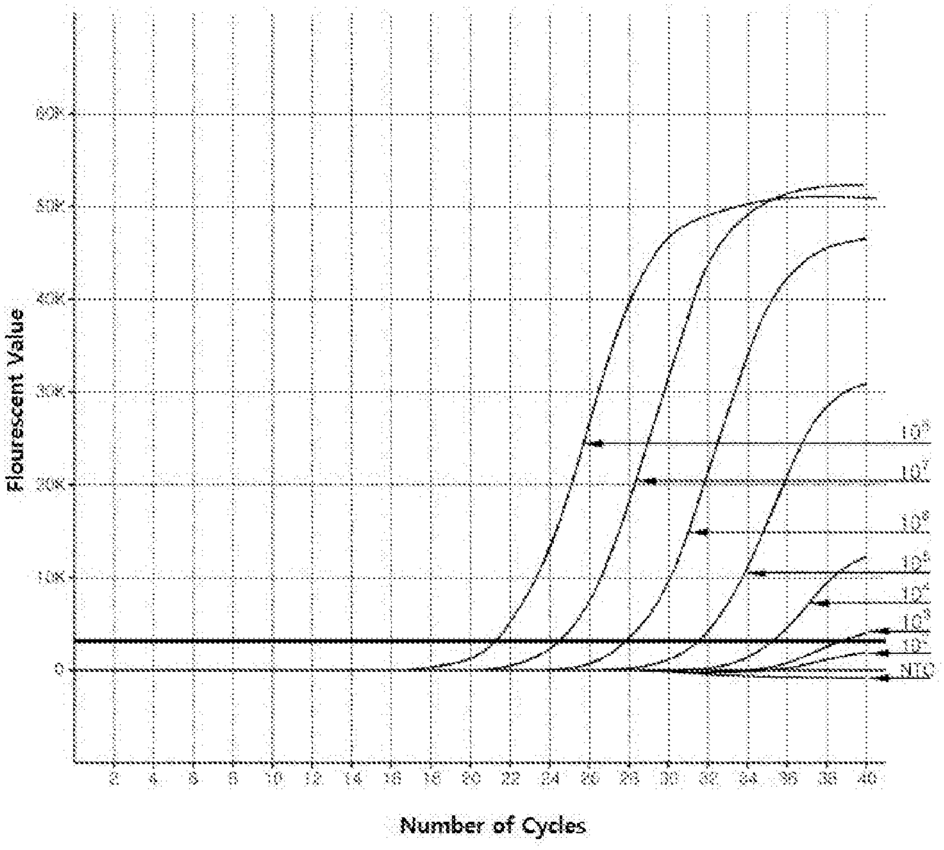

Flourescent Value
60K
50K
40K
30K
20K
10K
0
2 4 6 8 10 12 14 16 18 20 22 24 26 28 30 32 34 36 38 40
Number of Cycles
$10^8$
$10^7$
$10^6$
$10^5$
$10^4$
$10^3$
$10^2$
NTC

【FIG. 7】

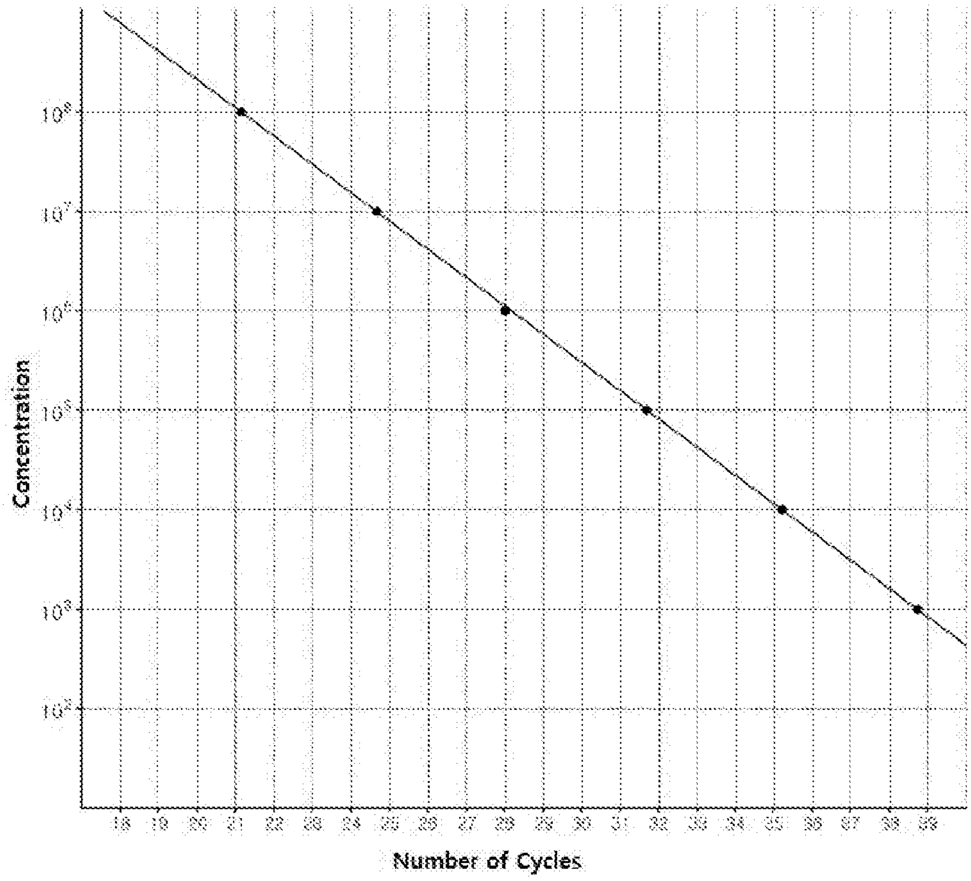

【 FIG. 8】

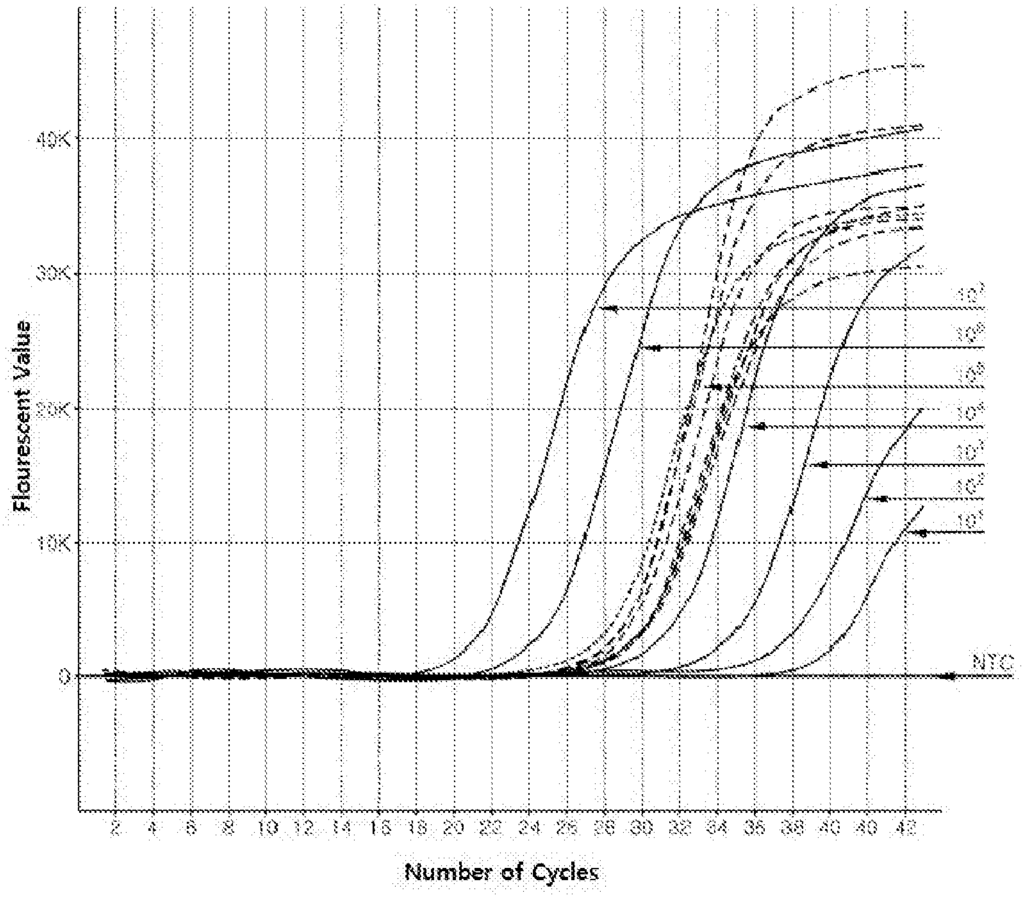

【 FIG. 9】

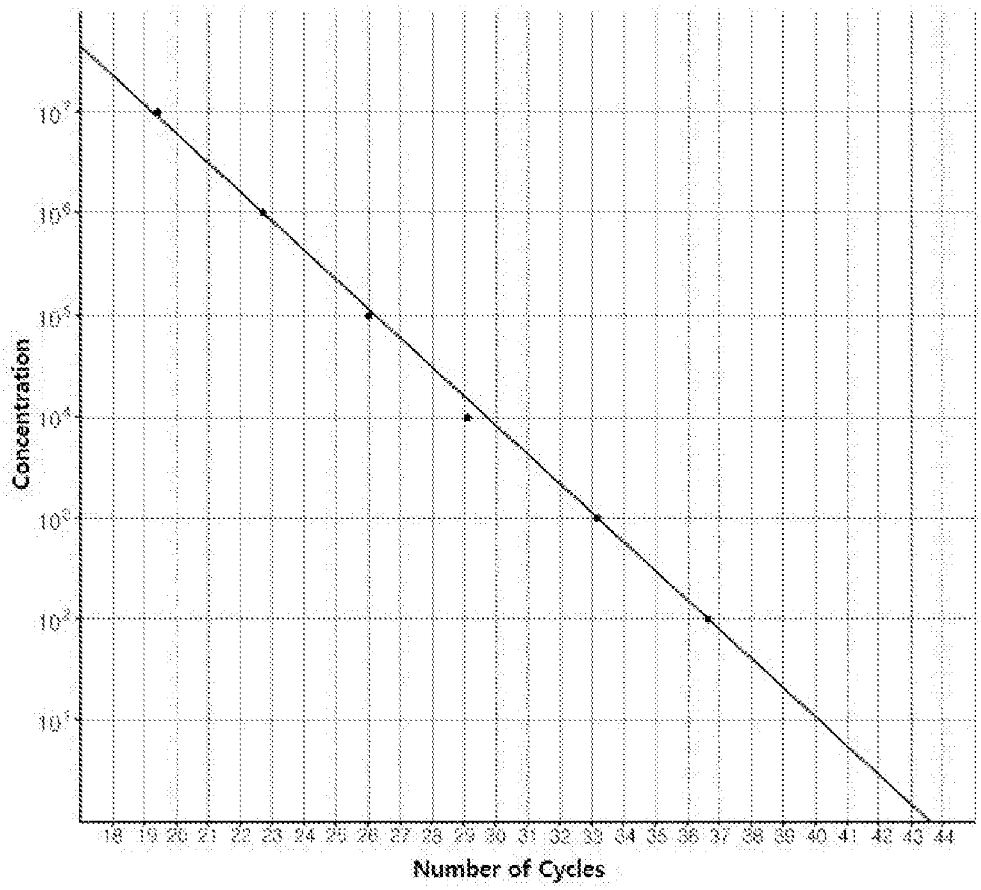
Concentration
$10^7$
$10^6$
$10^5$
$10^4$
$10^3$
$10^2$
$10^1$
18 19 20 21 22 23 24 25 26 27 28 29 30 31 32 33 34 35 36 37 38 39 40 41 42 43 44
Number of Cycles

【FIG. 10】

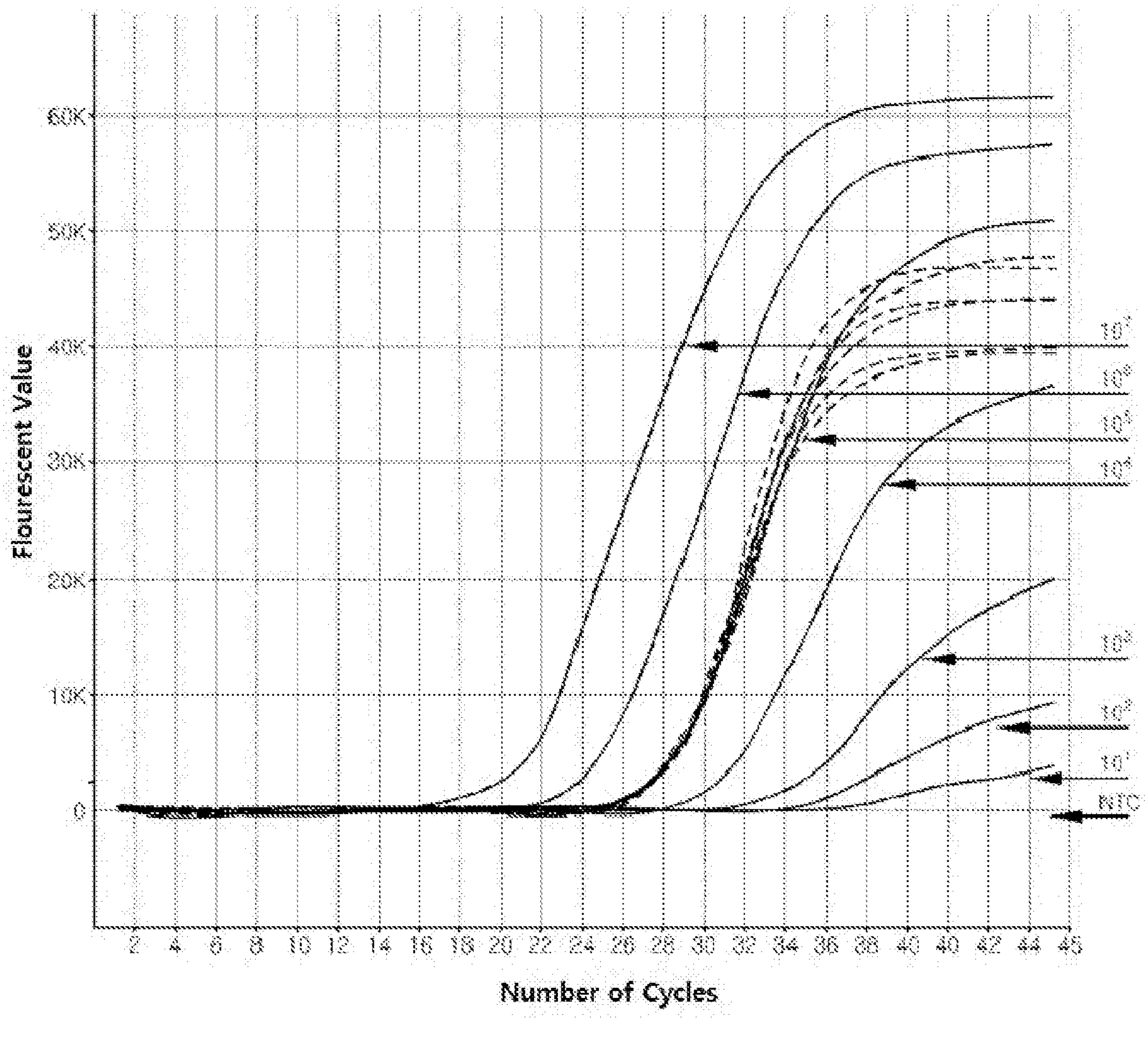
60K
50K
40K
30K
20K
10K
0
Flourescent Value
2 4 6 8 10 12 14 16 18 20 22 24 26 28 30 32 34 36 38 40 40 42 44 45
Number of Cycles
$10^7$
$10^6$
$10^5$
$10^4$
$10^3$
$10^2$
$10^1$
NTC

【FIG. 11】

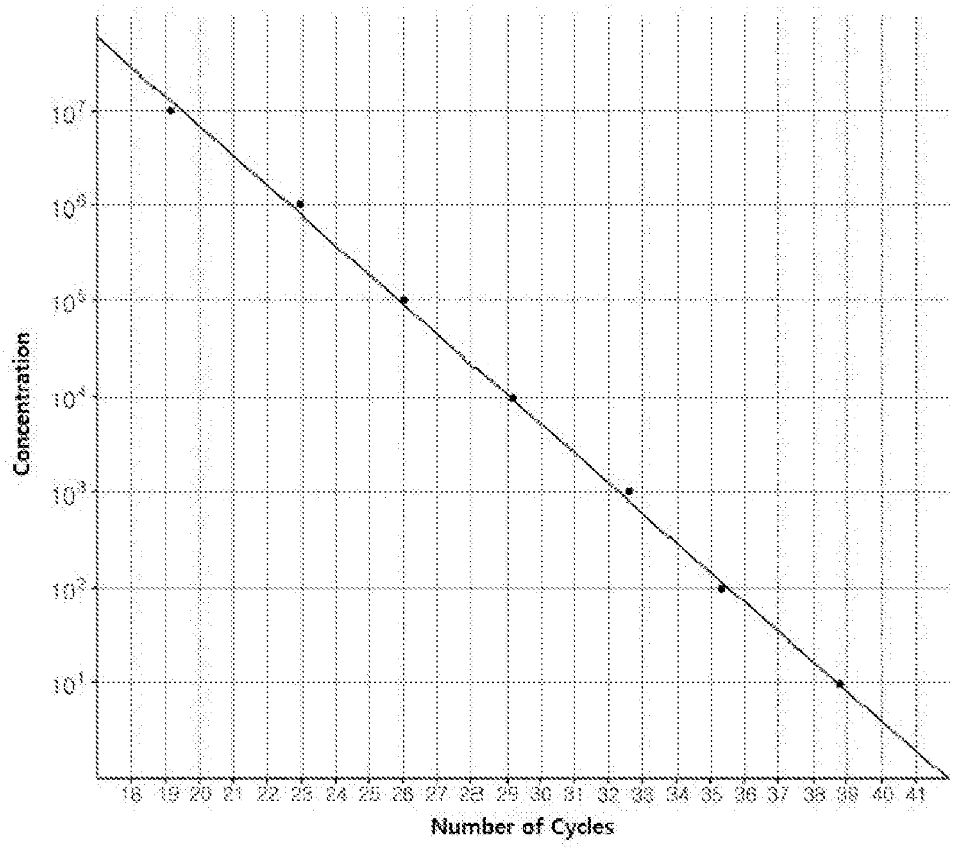
Concentration
$10^7$
$10^6$
$10^5$
$10^4$
$10^3$
$10^2$
$10^1$
18 19 20 21 22 23 24 25 26 27 28 29 30 31 32 33 34 35 36 37 38 39 40 41
Number of Cycles

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20010039014 A
- KR 148239
- US 5702590 A
- US 5647994 A
- EP 0691541 A
- US 5336760 A
- US 5897783 A
- US 6187270 B
- KR 1020080032904
- KR 794703
- KR 2008064558 W
- KR 10751036
- KR 20090020913
- KR 20100021532
- KR 20100002027


### Non-patent literature cited in the description


- *NBiochem. Biophys. Res. Commun.,* 1998, vol. 248, 200-203